Europäisches Patentamt

European Patent Office

Office européen des brevets

(19)

(11) EP 1 665 996 A2

(12) **EUROPEAN PATENT APPLICATION**

(43) Date of publication:
 07.06.2006 Bulletin 2006/23

(51) Int Cl.:
 *A61B 18/20* (2006.01)     *A61B 18/18* (2006.01)

(21) Application number: 05077934.7

(22) Date of filing: 22.02.2002

(84) Designated Contracting States:
 AT BE CH CY DE DK ES FI FR GB GR IE IT LI LU
 MC NL PT SE TR

(30) Priority: 02.03.2001 US 272745 P

(62) Document number(s) of the earlier application(s) in
 accordance with Art. 76 EPC:
 02748360.1 / 1 365 699

(71) Applicant: Palomar Medical Technologies, Inc.
 Burlington, MA 01803 (US)

(72) Inventors:
 • Altshuler, Gregory B.
  Wilmington
  Massachusetts 01887 (US)
 • Inochkin, Mikhail
  St. Petersburg 197198 (RU)
 • Khramov, Valery Yu
  St. Petersburg 195276 (RU)
 • Biruchinsky, Sergey B.
  St. Petersburg 197135 (RU)
 • Erofeev, Andre
  North Andover
  Massachusetts 01845 (US)
 • Belikov, Andre V.
  St. Petersburg 198217 (RU)

(74) Representative: Marlow, Nicholas Simon
 Reddie & Grose
 16, Theobalds Road
 London WC1X 8PL (GB)

Remarks:
 This application was filed on 20 - 12 - 2005 as a
 divisional application to the application mentioned
 under INID code 62.

(54) **Apparatus and method for photocosmetic and photodermatological treatment**

(57) This invention relates to apparatus for performing a treatment on tissue, comprising: a hand piece having a waveguide 5 with an output end for applying radiation from an optical radiation source to the tissue; and a mask coupled to the hand piece, the mask having a spatial pattern to allow passage of radiation to provide separate treatment spots in the tissue.

Fig. 1

EP 1 665 996 A2

**Description**

<u>Related Application</u>

This application claims priority to U.S. Provisional Application Serial No. 60/272,745 filed March 2, 2001 entitled *Apparatus and Method for Photocosmetic and Photodermatological Treatment.*

<u>Field of the Invention</u>

This invention relates to cosmetic and dermatological treatment using light, and more particularly to improved methods and apparatus for such treatment.

<u>Background</u>

Optical radiation has been utilized for many years in medical and non-medical facilities to treat various medical and cosmetic dermatology problems. Such problems include, but are by no means limited to, removal of unwanted hair, treatment of spider veins, varicose veins and other vascular lesions, treatment of port wine stains and other pigmented lesions, treatment of psoriasis, skin resurfacing and skin rejuvenation for treatment of wrinkles, treatment for acne, various treatments for reduction or removal of fat, treatment for cellulite, tattoo removal, removal of various scars and other skin blemishes and the like. Both coherent light, generally from a laser, and incoherent light, generally from a flash lamp or other lamp, have been used in such treatments.

In recent years, increasing interest in this field has centered on the use of incoherent light from various lamps both because of the potential lower cost from the use of such sources and because such sources are considered safer, both in terms of potential thermal or other damage to the patient's skin in areas overlying or surrounding the treatment area and in terms of eye safety. However, existing lamp-base dermatology systems have not fully realized either their cost or safety potential. One reason for this is that, even the best of these devices, have no more than a 15% efficiency in delivering the radiation generated to the treatment area. This means that larger and more expensive optical sources must be utilized in order to achieve energy levels required for various treatments. The energy lost in such devices can also produce heat which must be effectively removed in order to prevent thermal damage to the system, to permit applicators to be comfortably and safely held and to avoid thermal damage to the patient's skin. Apparatus for facilitating heat management also adds to the cost of these devices.

One potential source of thermal damage to the patient's skin in the use of these devices are local hot spots in the radiation beam being applied to the patient's skin. To avoid such local hot spots, it is desirable that the applied radiation be substantially uniform in intensity and in spectral content over substantially the entire beam. This has frequently not been true for existing lamp systems.

Another important factor in achieving both efficiency and safety is to optimize the lamp parameters, including the wavelength band or bands utilized, the intensity and the duration of radiation application for each particular treatment. Improved mechanisms for filtering of the lamp output to achieve selected wavelengths, for cooling the apparatus and for generating and controlling the radiation could further contribute to enhanced efficiency, reduced costs and greater safety.

A need therefore exists for improved apparatus and methods for the utilization of noncoherent radiation from a suitable lamp or other source to perform various medical and cosmetic dermatology treatments.

<u>Summary of the Invention</u>

In accordance with the above, this invention provides an apparatus utilizing a lamp for treatment of a patient's skin. The apparatus including a waveguide adapted to be in optical contact with the patient's skin and a mechanism for directing photons from the lamp to the waveguide to the patient's skin, which mechanism includes a sub-mechanism which inhibits the loss of photons from the apparatus. The mechanism may include a reflector, the reflector and waveguide being sized and shaped so that they fit together with substantially no gap therebetween. To the extent there is a gap between the reflector and waveguide it may be substantially sealed with a reflective material. The reflector is preferably sized and mounted with respect to the lamp so as to minimize the number of reflections for each photon on the reflector, the reflector preferably being small enough and mounted close enough to the lamp to achieve such minimum number of reflections. The reflector may be formed on an outer surface of the lamp. A tube may be provided surrounding the lamp with a gap between the lamp and the tube through which fluid is flowed to cool the lamp. The reflector may be formed on the inner or outer surface of the tube. The reflector is preferably cylindrical in shape. The reflector may be a scattering reflector and may include a mechanism for controlling the wavelengths filtered thereby. Alternatively, the reflector may be formed of a material which filters selected wavelengths of light from the light impinging thereon.

For some embodiments, there may be a gap between the reflector and the waveguide, a second reflector being mounted in said gap which, in conjunction with the reflector directs substantially all photons from the lamp to the waveguide.

The apparatus may also include a mechanism for selectively filtering light from the lamp to achieve a desired wavelength spectrum. This filtering mechanism may be included as part of one or more of the lamp, a coating formed on the lamp, a tube surrounding the lamp, a filter device in a gap between the lamp and the tube, a reflector for light from the lamp, the waveguide, and a filter device between the lamp and waveguide. The filtering mechanism may be an absorption filter, a selectively reflecting filter and a spectral resonant scatterer. The filter may include a multilayer coating.

The waveguide may be of a length selected to enhance uniformity of the light output from the lamp. The light output from the lamp may have resonances as a function of waveguide length, the waveguide preferably being of a length which is equal to one of the resonant lengths. The length of the waveguide is preferably greater than the smaller of the width and depth of the waveguide at its end adjacent the lamp.

The apparatus also may include a mechanism for controlling the angular spectrum of photons within the patient's skin. More specifically, a gap may be provided between the lamp and the waveguide which gap is filled with a substance having a selected index of refraction. Where a tube surrounds the lamp, this gap is between the tube and the waveguide. The length of the gap should be minimized and for preferred embodiments, the gap is filled with air.

The waveguide may have a larger area at a light receiving surface than at a light output surface and may have curved sides between these surfaces. The waveguide may also have a plurality of cuts formed therethrough, the cuts being adapted to have coolant fluid flowed therethrough. The waveguide may also have a surface in contact with the patient's skin which is patterned to control the delivery of photons to the patient's skin. The waveguide may also have a concave surface in contact with the patient's skin, which surface may be achieved by either the waveguide itself having a concave surface or a rim surrounding the surface having a concave edge. The depth of the concave surface is preferably selected to, in conjunction with pressure applied to the apparatus, control the depth of blood vessels treated by the apparatus. A mechanism may also be provided for detecting the depth of blood vessels in which blood flow is restricted by application of the concave surface under pressure to the patient's skin, this mechanism permitting pressure to be controlled to permit treatment of the vessels at a desired depth. Alternatively, the waveguide may have a skin contacting surface shaped to permit the application of selective pressure to the patient's skin to thereby control the depth at which treatment is performed. The waveguide may also be at least in part a lasing or a superluminescent waveguide and may include a lasing waveguide inside an optical waveguide. Alternatively, a lasing or superluminescent material may surround the lamp, photons from the lamp being directed to this material.

A mechanism may also be provided which delivers a cooling spray to both the patient's skin and the skin contacting surface of the waveguide just prior to contact. The waveguide may include a lower portion adjacent the patient's skin of a material which is a good conductor of heat and an upper portion of a material which is not a good conductor of heat, the thickness of the lower portion controlling the depth of cooling the patient's skin. Such control of cooling depth in the patient's skin may also be achieved by controlling the thickness of a plate of a thermally conductive material having a cooling fluid flowing over its surface opposite that in contact with the patient's skin. A detector may also be provided which indicates when the apparatus is within a predetermined distance of the patient's skin, the cooling spray being activated in response to such detector.

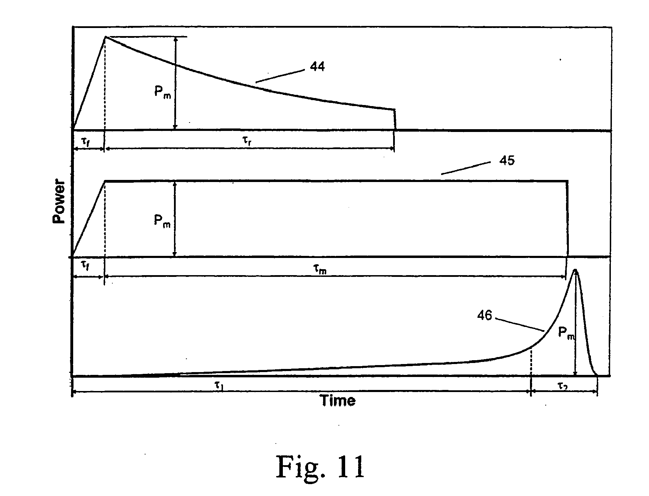

The apparatus may also include rearward facing light output channel from the waveguide which leads to a backscattered detector, the channel being at an angle $\alpha$ to a perpendicular to the skin that only backscattered light reaches the detector. The lamp may be driven with a power profile which is one of the power profiles 44, 45 or 46 of Fig. 11. The waveguide may be formed as a unitary component with the lamp passing through an opening formed therein.

The invention also includes methods for utilizing the lamp to perform various treatments on a patient's skin including:

> a method for performing hair removal utilizing the parameter of Table 1;
> A method for performing treatment vascular lesions utilizing the parameters of Tables 2, 3 and 4;
> A method for performing skin rejuvenation utilizing the parameters of Tables 2 and 6;
> A method for treating acne by killing bacteria, thermolysis of the sebaceous gland and/or killing spider veins feeding the sebaceous gland; and
> Treating pigmented lesions utilizing the parameters of Table 5.

The optimum spectrum for the optical radiation from the lamp supplied to the patient's skin is such that the ratio of the temperature at the treatment target to the temperature of the patient's epidermis is a selected value S, which is preferably greater than 1. Filtering may be used so as to provide one or more wavelength bands from the lamp output to achieve the above objective. A waveguide may be utilized having scattering properties which are dependent on waveguide temperatures and this feature may be utilized automatically to protect the patient's skin. A reflecting absorbing or phase mask may be mounted or formed at the end of the waveguide to control regions of the patient's skin to which radiation is applied.

The foregoing and other objects, features and advantages of the invention will be apparent from the following more particular description of preferred embodiments of the invention as illustrated in the accompanying drawings, like elements in the various figures having the same or related reference numerals.

Brief Description of the Drawings

Fig. 1 and Fig. 2 are a cut-away side view and a longitudinal cross-section view respectively of a lamp device for an embodiment of the present invention;

Fig. 1a and Fig. 2a are a cut-away side view and a longitudinal cross-section view respectively of a lamp device for another embodiment of the present invention;

Fig. 3 is a chart showing the absorption spectra for certain natural chromophores;

Fig. 4 is a chart of penetration depth spectra for different types of skin;

Fig. 5 is a chart showing typical arc-lamp emission spectra for selected parameters;

Figs. 6a and 6b are charts of temperature rise for the hair shaft and for the hair matrix relatively to temperature rise of the basal layer for white skin and dark skin respectively;

Figs. 7a - 7c are charts of initial lamp spectra and profiled spectra for different skin types and/or treatments;

Fig. 8 is a chart illustrating the dependencies of light illumination at 1mm depth and 3mm depth relative to illumination of the epidermis on the size of the light beam;

Fig. 9a and Fig. 9b are charts illustrating the distribution of light on the surface and at depth for a 10mm beam width and 15mm beam width respectively;

Fig. 10 is a chart illustrating the dependence of fluence improvement due to photon recycling on beam width.

Figs. 11a - 11c are diagrams of pulse power over time for three different pulse shapes.

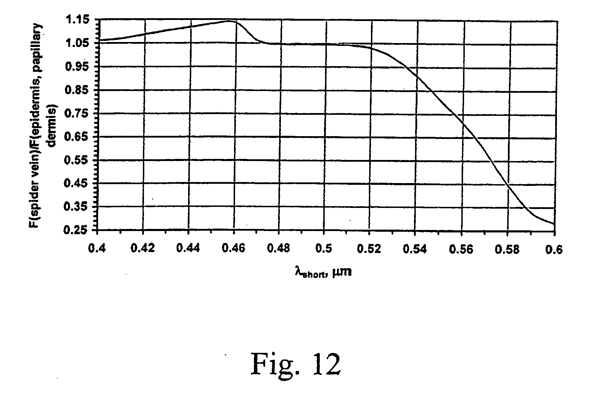

Fig. 12 is a chart illustrating the relationship of wavelength in micrometers to the ratio of fluids at a shallow target (spider vein) to fluids at the epidermis.

Fig. 13a - 131 are schematic representations of various lamp cross-sections suitable for use in practicing certain aspects of the invention.

Figs. 14a and 14b are front cutaway views of lamps for alternative embodiments having different filter configurations.

Fig. 15a and 15b are perspective views of two alternative waveguide configurations suitable for use in practicing the teachings of this invention.

Fig. 16 is a perspective view of still another waveguide suitable for use in practicing the teachings of the invention.

Fig. 17 is a chart illustrating the dependence of the angular spectrum of the photons on the material placed between the outer tube of the lamp and the waveguide.

Fig. 18 is a side cutaway view of a lamp in accordance with an alternative embodiment of the invention wherein waveguide material substantially surrounds the lamp.

Fig. 19 is a chart illustrating the dependence of radiation uniformity on waveguide length.

Figs. 20a - 20d are side views (cutaway from Fig. 20c) of various waveguides suitable for use in practicing the teachings of this invention for different applications.

Fig. 20e is a bottom view of a waveguide having a mask formed thereon.

Figs. 21a and 21b are side views of lamp configurations utilizing waveguides with lasing or superluminescent properties.

Fig. 22 is a chart illustrating the output spectrum for a lamp with a standard waveguide and an illustrative output spectrum for a lamp having a lasing or superluminescent waveguide of Fig. 21.

Figs. 23a and 23b are side cutaway views for two alternative embodiments incorporating novel filtering techniques.

Fig. 24 is a perspective view of a waveguide having novel cooling channels formed therethrough.

Fig. 25 is a side view of a waveguide embodiment exhibiting unique cooling capabilities.

Fig. 26 is a side view of still another mechanism for cooling a waveguide.

Fig. 27 is a side view of still another cooling mechanism for a waveguide; and

Fig. 28 is a semi-schematic partially cutaway front view of an embodiment of the invention which provides a unique mechanism for detecting safe irradiation of a patient's skin.

Detailed Description of Preferred Embodiments

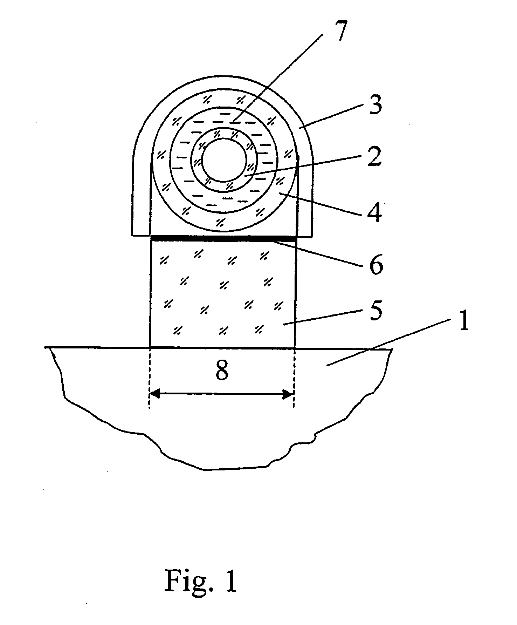

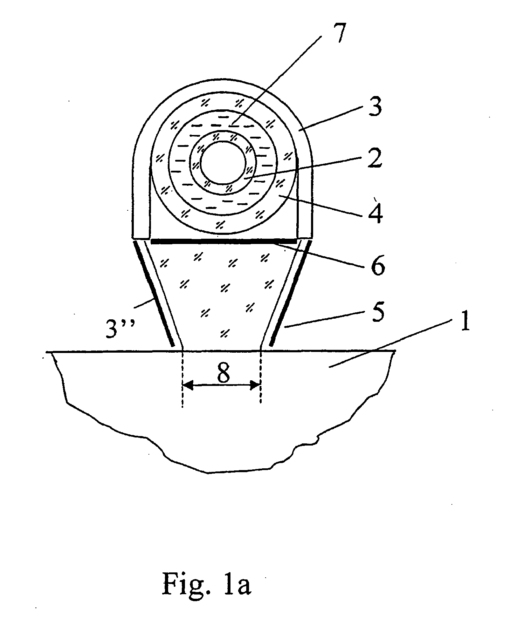

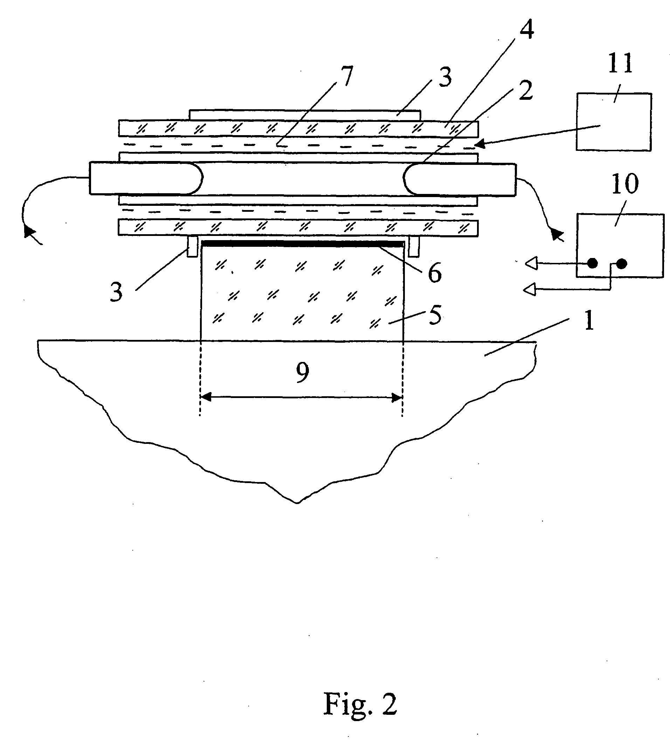

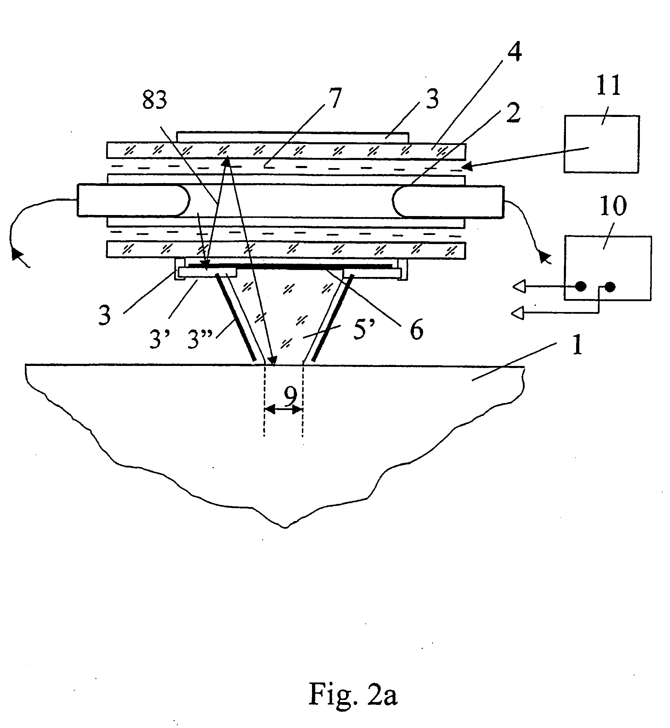

In Fig. 1 and Fig. 2, cross-sections of an illustrative device D for cosmetic and medical dermatological treatment of the skin 1 are shown; while most of the following discussion will be with respect to this device, this is not a limitation on the invention.

The light source is represented by a linear tubular arc lamp 2 filled with a gas (Xe, Kr, Hg etc.) which lamp is enclosed in a glass or crystal tube 4 with cylindrical cross section. The gap 7 between the lamp 2 and the tube 4 is filled with liquid or gas which may be pumped. A reflector 3 is placed around the tube with or without gap. The reflector may include a vacuum or galvanic high-reflective coating on a substrate having a curved tubular part and extending flat parts which reach (and preferably overlap) a waveguide 5 on all sides. The reflector includes end-plates 3, which are best seen in Fig. 2, and which function to minimize any gap between reflector 3 and waveguide 5. To the extent any gap remains, it may be filled with a reflective material to minimize photon leakage. The reflector should also be made in a way such that gaps between the reflector and the waveguide are minimized, not exceeding 10% of the total reflector surface, and that the reflection index is close to 1.00 for all wavelengths of radiation impinging thereon, and preferably not being less than 0.85 for any such wavelength. The reflector may be in the form of a thin flexible metal sheet with a reflecting surface facing the lamp. The reflecting surface may be a high-grade polished surface or may have a high-reflection coating. The coating may for example be silver or gold. The coating may be covered by a protective polymer film or thin non-organic dielectric in order to protect the coating against chemical degradation. The reflector coating may be a diffuse reflecting coating or a layer of powder (for example, $BaSO_4$) with low absorption in the spectral range of radiation used for skin treatment.

The reflector is optically coupled with waveguide 5. Direct light from lamp 2 and light from lamp 2 reflected by reflector 3 are coupled through filter 6 and the waveguide for delivery to the skin. The waveguide may be made of a glass or dielectric crystal. The radiation spectrum of the lamp may be converted into a spectrum which is optimum for treatment of the selected target in the skin, this transformation of the spectrum being provided by one of the following techniques, or a combination thereof: (a) absorption in the envelope of the lamp 2, (b) absorption in the liquid in gap 7, (c) absorption in tube 4, and/or (d) absorption or directed scattering in filter 6. Energy absorbed in the envelope of lamp 2, in the liquid in gap 7 and/or in tube 4 may be converted to a desired wavelength spectral range as a result of Stokes luminescence. For example, tube 4 may be of a florescent material or a liquid doped with dye may be employed in gap 7. These may act as a high-pass filter, fluorescing above a selected cut-off wavelength to move energy from blue to red. This may provide some protection for the epidermis without energy loss. Both converted radiation and unconverted radiation from the lamp may be delivered to the skin through waveguide 5.

Absorption may be provided by doping the above-mentioned components with, for example, ions of metals such as, Ce, Sm, Eu, Er, Cr, Ti, Nd, Tm, Cu, Au, Pt, organic and/or inorganic dyes, for example semiconductor microcrystals, or other suitable doping substances dissolved in liquid or glass. Filter 6 may be made as a multilayer dielectric interferometric coating on the surface of waveguide 5, on a transparent substrate or on a scattering medium. The scattering medium may be made as a special regular profile on the surface of waveguide 5 produced, for example, by photolithography. It can, for example, be a phase grating with spectral and angle transmission needed for treatment. Filter 6 may also be several stacked filter components, each filter working within a selected band or bands, some of which may be relatively narrow. Using several filters makes it easier to get a desired wavelength and, by using several filter components, no one filter component heats excessively. To the extent filtering is done by coatings on for example tube 4 and/or reflector 7, such coatings may also be multilayer.

Filter 6 can also be a cold or nonabsorption filter, which preferably has multiple layers, for example 30 layers. Such filters selectively reflect at the various layers creating interference which can eliminate undesired wavelengths. The reflected radiation can also be optically removed. However, while these so-called multilayer dielectric filters are advantageous in reducing heat management problems, they are generally not as effective in eliminating short wavelengths, and while filtering light very well for collimated beams, for high divergence lamp beams, they cannot provide the sharp cut off filtering needed for better wavelength selectivity. Other filters which might potentially be used as filter 6 include a film of a semiconductor material having an absorption band which is a function of an electric field applied thereto. Such semiconductor film may experience a Stark effect, wherein the cutoff frequency may be controlled by controlling a current or voltage passed through the material.

Scattering filters may also be used for the filter 6. Such filters may for example be formed of liquid crystal material, and electric current or field applied across the material controlling the wavelength where the refractive index of the components are the same, there being no scattering for such wavelengths permitting photons at these wavelengths to pass therethrough. Other wavelengths are attenuated by scattering. A scattering filter 6 can be multilayered with different materials or different materials can be used in a single layer of liquid crystal material to control the width and wavelength of the passband. Such passband would typically be both temperature and electric field dependent. Such a scattering filter should be designed to primarily scatter undesired wavelengths in large angle, including backwards. The large angle of the backscattered beam results in multiple reflections which further attenuate these unwanted frequencies. Finally, an additional filter 2 may be mounted in channel 7 so that the filter is also cooled by the coolant in this channel. Other options, either currently known or developed in the future for both the location and type of filter used to achieve a desired output wavelength band from device D may also be employed. There are three criteria which are important in selecting the location or locations for the filters and the type of filters utilized to achieve a desired output wavelength band from device D. These criteria are thermal design, the selection and positioning of the filter so as to minimize heat generated therein and/or to facilitate the removal of the heat therefrom. The second criteria, which is particularly important for the safety and efficacy of the treatment, is the sharpness of the signal cut-off for the full angular spectrum of the lamp. The third criteria is high transmission of the wanted wavelengths. Filtering removes some of the energy of the beam and the more of this energy which is dissipated as heat in absorption filters, the lower the efficiency of device D.

Wave guide 5, at least during a treatment, is in optical and thermal contact with skin 1 of the patient in order to provide efficient coupling of light into the skin and cooling of the skin surface. For low mean power of the lamp (including low repetition rate of the treatment), cooling of the device components (lamp 2, reflector 3, absorbing filters) can be provided by natural convection. For high mean power of the lamp, additional cooling may be provided by a cooling system 11 (Fig. 2) flowing a liquid or gas through, for example, channel or gap 7, cooling in this case resulting from thermal contact of the cooled components with the flowing cooling agent, for example the liquid in gap 7. If cooling of the skin (epidermis) is necessary, waveguide 5 may be cooled before, during and/or after irradiation. Exemplary techniques for cooling waveguide 5 will be described below. Lamp power supply 10 provides the necessary power, duration and shape of lamp emission pulse for optimum irradiation of the skin target. An example of a suitable power supply is provided in co-pending application serial # 09/797,501, filed March 1, 2001. The optical layout of device D provides minimum losses of light and maximum reflection index for reflector 3 and the walls of the waveguide. Therefore, maximum efficiency in the utilization of energy from the lamp is obtained, permitting the cost of the device to be minimized. Photons reflected from the skin pass into device D through waveguide 5 and are directed back to the skin by reflector 3 and waveguide 5 with maximum efficiency, resulting in increased irradiation of the target in skin 1. These photons generally pass through lamp 2 with minimal loss of energy. This further increases the efficiency of energy utilization, permitting a further decrease in required lamp output, and thus in the cost of the device.

The optical system described above may sometimes be referred to as the optical system of skin irradiation with minimum photon leakage (MPL). The optical system of device D should also provide a relatively large spot size 8, 9 for the light beam on the surface of the skin 1, maximum uniformity of light intensity on the skin surface in order to decrease the possibility of epidermal damage and optimum light distribution for the destruction of a target inside the skin. Thus, in defining the parameters of the device, it is necessary to define parameters providing: 1) the desired spectrum of light to be delivered to the skin, 2) the size of the light beam on the surface of the skin with maximum uniformity of its spatial distribution, 3) optimum distribution of the light inside the skin, and 4) a desired fluence, duration and the temporal shape of the light pulse delivered to the skin. Conditions (1)-(4) depend on the selected target (blood vessel, hair follicle, dermis, etc.) and the patient's skin type. These conditions are considered taking into account the distribution of lamp light in the skin and the theory of selective photothermolysis (Anderson RR, Parrish J.; Selective photothermolysis: Precise microsurgery by selective absorption of the pulsed radiation. Science 1983; 220: 524-526) and extended theory of selective photothermolysis (Altshuler G.B., Anderson, R.R., Zenzie H.H., Smirnov M.Z.: Extended Theory of Selective Photothermolysis, Lasers in Surger and Medicine 29:416-432, 2001).

Figs. 1a and 2a illustrate an alternative embodiment of the invention suitable for use where greater fluence is desired from a given lamp and a smaller spot size is either desired, or at least acceptable. Such a result would for example be acceptable where the treatment is at shallower depths rather than treatments at deeper depths. The desired results are achieved by using a concentrator waveguide 5' in place of the waveguide 5, waveguide 5' having walls which angle in so that the skin-contacting surface of the waveguide is smaller then the light-receiving side of the waveguide. However, while the straight walled waveguide 5 has substantially total internal reflection of photons therein, the angled walls of concentrator waveguide 5' permit some photon leakage through these walls or facets. To prevent photon loss as a result of this leakage, a reflector 3" is provided adjacent each such wall, for example being coated on the wall, which reflector has high reflection, for example greater than 95%. Both recognition of the waveguide leakage problem and the use of reflectors 3" or a comparable external reflector are considered novel and part of the invention.

Fig. 2a also illustrates another novel feature of this embodiment which compensates for the fact that lamp 2 may be longer then the length of the desired spot size. Normally this would result in photon leakage and the loss of photons. However, in Fig. 2a, reflectors 3' are provided in the gap between reflector 3 and waveguide 5' which reflectors are effective to couple rays or photons 83 from end portions of the lamp through waveguide 5' to the patient's skin. This embodiment thus result in a roughly 50% increase in the fluence improvement achieved by use of a concentrator waveguide.

The Propagation and Absorption of Lamp Light in the Skin

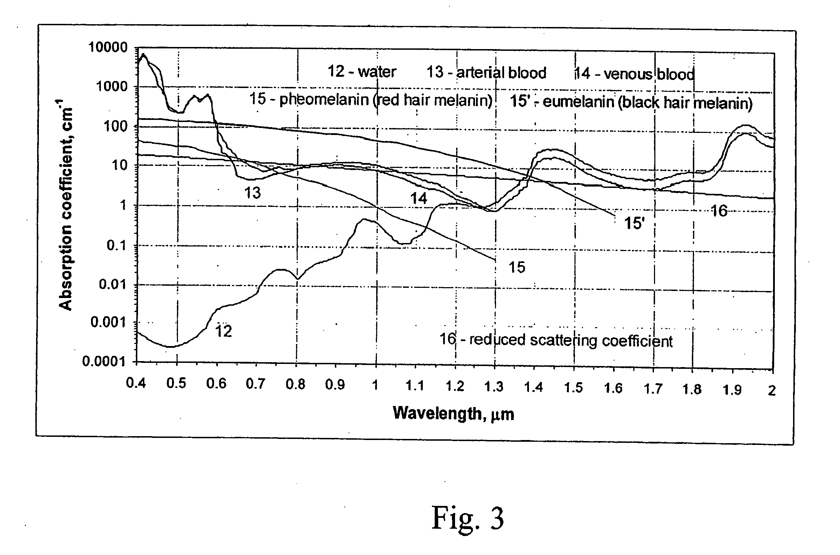

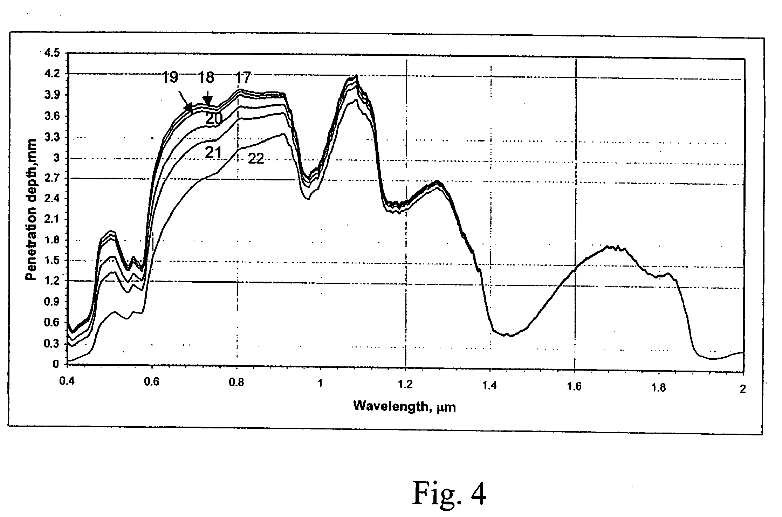

Differences in the propagation and absorption of lamp light as opposed to laser light in the skin results at least in part from differences in their selected range, the lamp spectrum being very wide (200-1000 nm), which is thousands to tens of thousands times wider than the spectral range of laser radiation. The angular spectrum of a lamp source may be as wide as $\pm 180°$. That is hundreds to thousands times wider than the angular spectrum of laser radiation. Therefore the propagation and absorption of lamp light in the skin differ considerably from that of a laser. In the near UV, visible and near IR ranges, the absorption of water, hemoglobin, oxyhemoglobin, melanin, lipid and protein, as well the absorption of dopants (carbon particles, molecules of organic and inorganic dyes), may be used for optical/ light therapeutic treatment of the skin. In Fig. 3, spectra are shown for the main natural skin components, namely 12-water, 13-arterial blood (95% hemoglobin, 5 % oxyhemoglobin), 14-venous blood (65% hemoglobin, 35% oxyhemoglobin), 15 - phemelanin (red hair), 15'-eumelanin ( dark hair, epidermis), 16 - reduced scattering coefficient of the skin. In Fig. 4, the depth dependences at which three times attenuation of a collimated wide light beam occurs as a function of wavelength is shown for different types of skin (17-white blond, 18-white brunet, 19-japanese, 20-indian, 21-mulatto, 22-african-american).

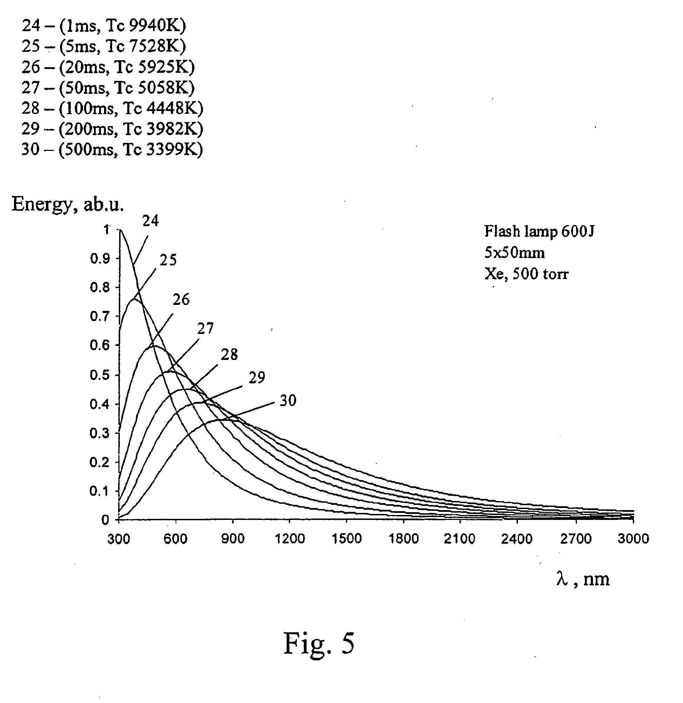

In Fig. 5, typical arc lamp emission spectra (without luminescent bands containing minor parts of the total energy) for different durations and equal energies of light pulse are shown. These curves are obtained for the same lamp having a 5x50mm discharge gap filled by Xe under a pressure of 450 torr with the following pulse durations: 24 - 1ms, 25 - 5ms, 26 - 20ms, 27 - 50ms, 28 - 100ms, 29 - 200ms, 30 - 500ms. The different pulse durations correspond to different color temperatures of the lamp which determines the shape of the lamp emission spectrum. Thus, as can be seen from Fig. 5, changing the pulse width can be used to shift both the output spectrum and the color temperature. As can be seen from Figs. 3, 4, and 5, the spectrum of the lamp covers the absorption bands of all chromophores in the skin; therefore the lamp can be use for all skin chromophores. However, in order to achieve optimum treatment and utilization of light energy, it is necessary to provide the correct combination of color temperature of the lamp, spectral filtering, size and divergence of the beam at the output of the waveguide, intensity, fluence, duration and temporal shape of light pulse. These conditions depend strongly on the type of therapy. The apparatus described in the present invention is intended mainly for cosmetic procedures and treatment of dermatological problems which influence cosmetic properties of the skin.

Among these procedures, the following are of particular interest: management of hair growth; treatment of vascular lesions and pigmented lesions; and improving skin structure including reducing wrinkles/ skin rejuvenation, coarseness, low elasticity, irregular pigmentation, inflammatory acne and cellulite.

Management of Hair Growth

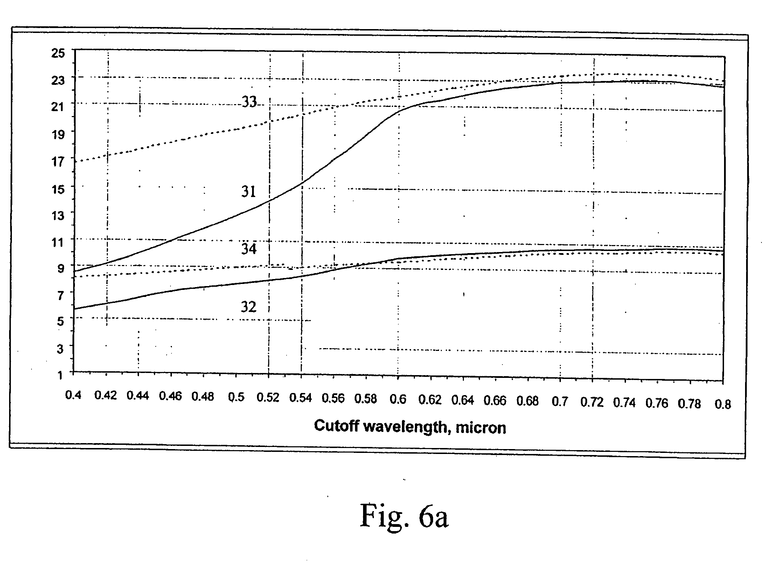

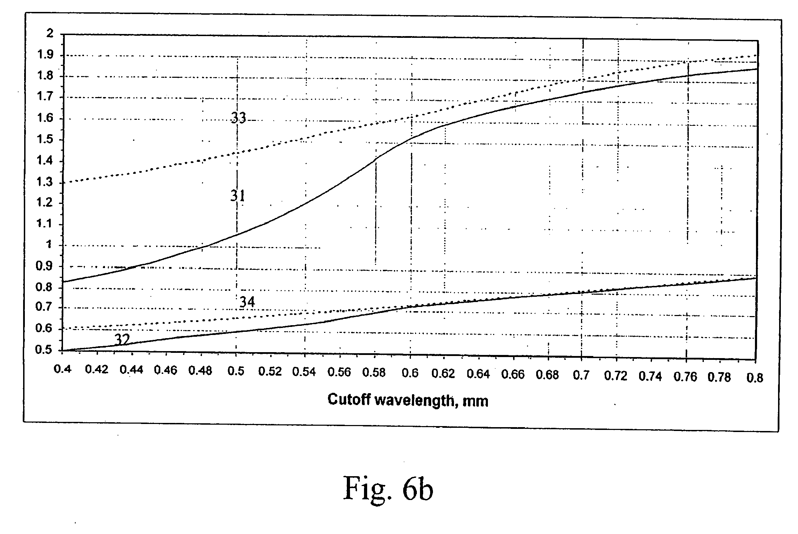

If selective, substantial damage to a hair bulb takes place, it becomes possible to stop or delay hair growth and to decrease hair size and pigmentation. Conversely, very light damage of the hair matrix can accelerate hair growth and pigmentation. Damage to follicle stem cells which are located in the outer root sheath at the level of the bulge can result in permanent hair removal. Permanent hair removal is also possible if dermis surrounding a hair follicle is damaged so that the follicle structure is fully or partially replaced by connective tissue, i.e., a microscar appears in place of the follicle. Photoepilation takes place due to the heating of follicles as a result of light absorbed by melanin contained in the hair matrix or hair shaft. The greatest concentration of melanin is in the hair matrix located inside the dermis or subcutaneous fat at a depth of 2-5 mm from the skin surface. Thus, in order to provide management of hair growth, the first damage targets are the hair bulb and the stem cells at the depth of the bulb which is approximately 1-1.7 mm from the skin surface, and a second damage target is the matrix located at 2 to 5 mm. A significant problem in hair growth management is preserving the overlying epidermis which also contains melanin. From Figs. 3, 4, 5, it can be concluded that, in order to provide selective damage of hair follicles, the radiation spectrum should be 360-2400 nm. The short-wavelength part of the spectrum is limited by potential damage to proteins, including DNA. The upper wavelength is limited by strong water absorption. Effective absorption of melanin takes place in the range of 360-1200 nm. However, a total cut-off of the 1200-2400 nm portion of the spectrum is not desirable because deeply penetrated infrared light is absorbed by water and provides additional, but not selective, heating of the hair follicle. In this case, the spectral components which are close to water absorption bands (Fig. 4) near 1.4 $\mu$m and 1.9 $\mu$m should be eliminated from the radiation spectrum because these wavelengths are absorbed in the epidermis and may cause overheating thereof, leading to patient pain and potential epidermal destruction. The best way to filter these wavelengths is to use water as a "water" spectral filter. In device D (Fig. 1, 2), filtering water is placed in the gap 7 between lamp 2 and tube 4. An appropriate thickness for this water to effect filtering is estimated to be within the range 0.5-3 mm. Since the absorption by melanin is basically within the range of 360-800 nm, the color temperature $T_C$ of the lamp should be within $T_C$=3000-10000 °K (Fig. 5). Filtering of short-wavelengths is determined by the type of the skin. In Figs. 6a,6b, the dependence of the ratio of the hair matrix (3mm depth) temperature to the temperature of the basal layer (31), and the dependence of a ratio of the hair shaft temperature at the depth of the bulge (1mm) to the temperature of the basal layer (32) on the wavelength of the short wavelength cut-off filter under fixed energy of lamp pumping are shown. The same dependences for pressed or cooled skin where blood is removed from small vessels in the dermis are shown by dotted curves (33,34). From Fig. 6a, it is seen that in the case of white skin, the use of short-wavelength radiation substantially increases the efficiency of stem cell destruction and the pressing or cooling of the skin causes the same result for the hair matrix. In this case, the thermal influence in the epidermis increases, but is lower in absolute value than in the pigmented hair shaft and hair matrix. In strongly pigmented skin (Fig. 6b), the short-wavelength cut-off should be raised. The dependence represented in Figs. 6a, 6b indicate the requirements for the filtering of short-wavelength radiation for different types of skin. This data is represented in table 1.

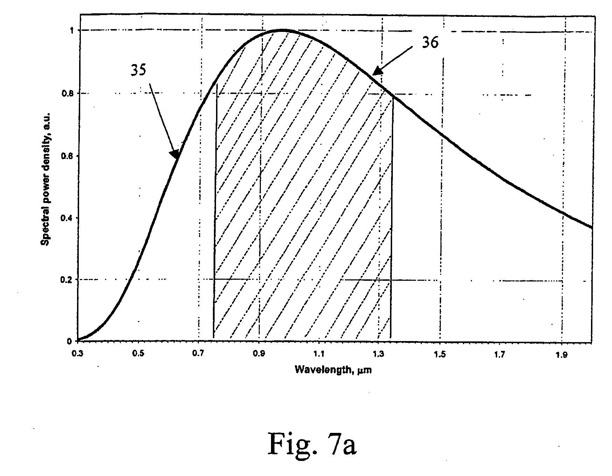

In Fig. 7a, the spectrum of the lamp under Tc = 5000°K (35) and after filtering (36) is represented. This spectrum is optimized for treatment on mulatto skin with brown-black hair. With this spectrum, maximum heating of the hair matrix without overheating the epidermis is achieved for a defined energy of lamp pulse. The upper or far wavelengths of the spectrum are filtered by a water filter in gap 7 of 1 mm thickness.

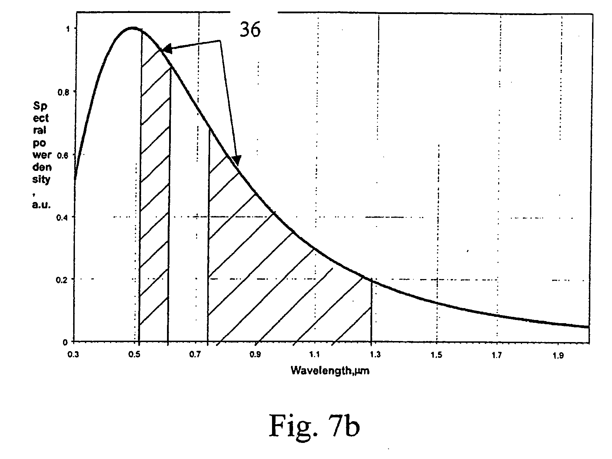

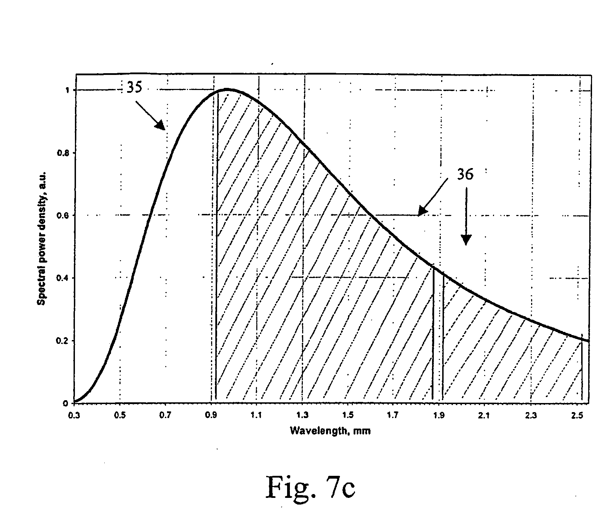

In Fig. 7b, the spectrum of the lamp for Tc = 6000°K before filtering(35) and after filtering (36) is represented. This spectrum is optimized for treatment of deep (0.3-1.0 mm depth) vascular. In Fig. 7c, the spectrum of the lamp for Tc = 3000°K before filtering (35) and after filtering (36) is represented. This spectrum is optimized for treatment of collagen due to water absorption.

The spectrums 36 shown in Fig. 7a-7c will each be referred to as a profiled spectrum of lamp [PSL]. The spectrum of the lamp is attenuated (profiled) for both the short and far or long wavelengths in order to provide maximum heating of the target while not overheating the epidermis. This condition can require several filtered bands (see spectra in tables 2-4). The optimum PSL for a given procedure may be one or more wavelength bands obtained, generally by filtering, from the output spectrum of the lamp, the band or bands being selected so that the ratio of the temperature rise of the target (hair shaft, matrix, vessel, vein, pigment lesion, tattoo, etc.) to the temperature rise of epidermis is more than a certain numbers S, which number S is dependent on from the level of safety for the procedure. The higher the number S, the higher the safety level. To maximize efficiency of the lamp, S should be about 1.

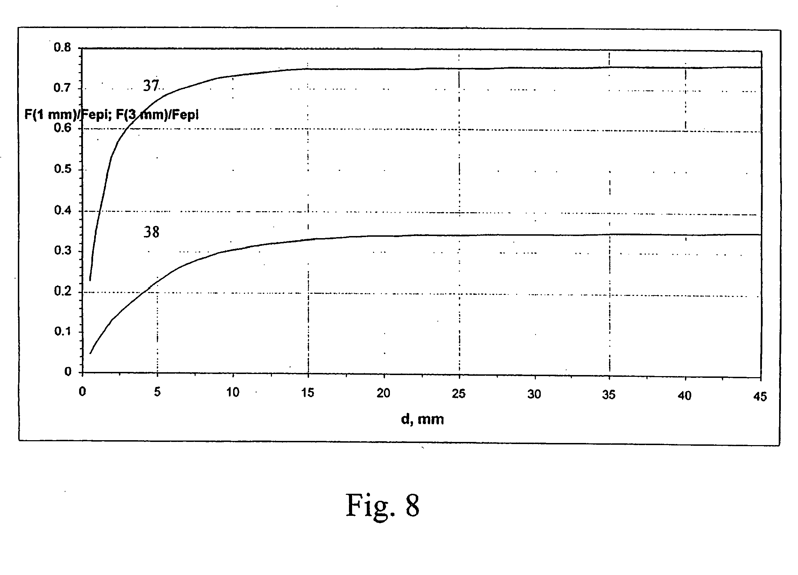

The dimensions of the beam are also important. It is known that for increasing beam size and constant intensity (fluence) on the surface, the intensity (irradiance) of light at depth increases and saturates once some transverse dimension of the beam is achieved (see Fig. 8).

When this dimension is increased, the ratio of illumination at a depth of 3-5 mm (where the hair bulb is located) to the illumination of the epidermis reaches a maximum, thus making it possible to provide maximum temperature at the hair bulb or stem cells with minimum risk of epidermal damage/destruction.

Fig. 8 shows the dependence of the ratio of the heat production on a melanin target in the skin at a depth of 1 mm (F =1 mm) (curve 37) and 3 mm (F=3 mm) (curve 38) to the heat production at the basal layer $F_{epi}$ with the same melanin concentration at the target for a lamp with color temperature $T_c$=6000K and the appropriate PSL on the size of the beam formed by the device D shown in Figs. 1, 2. The length 9 of the beam is fixed and equal to 45 mm. Usually this length is limited by the length of the lamp discharge gap. The width of the beam is varied within a range of 1-45 mm. Fig. 8 shows that for a deep target in the skin, the width of the beam should be more than 10 mm (minimum beam width d=10 mm). Best results are achieved when the width 8 is greater then 15 mm.

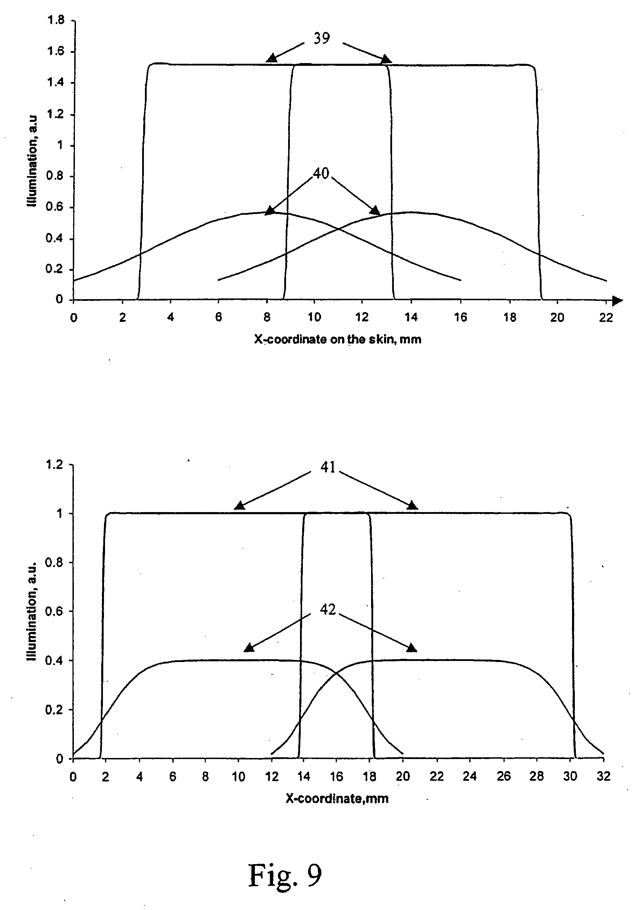

The second advantage of the wide beam is uniformity of illumination of the hair follicle at depth. For a beam of width <10 mm, the distribution at depth has a gaussian shape with sharp maximum. Therefore a large percentage overlapping of the beams when scanning along the skin is necessary for uniform irradiation of the follicles. This leads to a considerable decrease in the rate of treatment, decrease in efficiency of energy utilization and increase in the cost of the procedure. Further, the possibility of "missing" follicles because of the non-uniform overlapping, and hence the rapid growth of missed hair, still exists. The distributions of light intensity produced by device D for a beam of 10 mm (curves 39, 40) and 16 mm (curves 41, 42) are represented in Fig. 9. The curves 39 and 41 show the distribution on the surface and the curves 40 and 42 describe the distribution at depth. Fig. 9 shows that uniform overlapping of beams with 10 mm width needs at least 27% (Fig. 9) overlap whereas only 15% overlap is necessary for beams of 16 mm width.

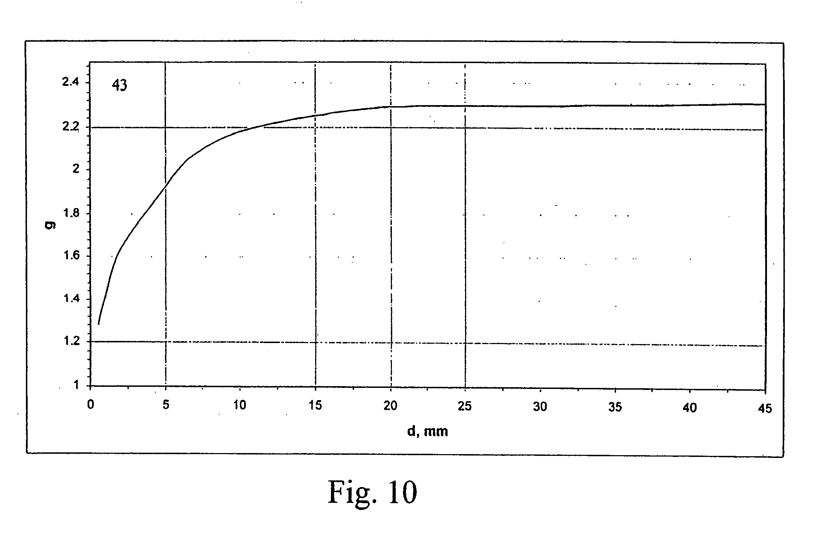

A third advantage of wider beams becomes apparent in lamp-based devices with an MPL optical system as is shown in Figs. 1, 2. As is discussed above, for these MPL systems, photons reflected from the surface are returned back to the skin and increase the utilization efficiency of the lamp energy. This effect may increase irradiation inside the skin up to three times, if the lamp-based devices with MPL optical system has very low leakage of photons. However it is greater if the size of the beam is increased. Fig. 10 shows the dependence 43 of skin irradiation amplification g caused by the return of the photons reflected from the skin on the size of the beam d for the same conditions as for Fig. 8. Fig. 10 shows that the effect of amplification is achieved if the beam width is >10 mm. Thus, the minimum dimensions of the beam for the hair management application is preferably about 10 mm, >15 mm being preferable. The requirements of pulse duration and temporal shape are now considered as well as intensity and light flow. In order to provide temporal injury or growth stimulation, critical parts of a follicle include the hair bulb, and more important the hair matrix, of a hair follicle in anagen stage. The thermal relaxation time of a hair matrix for a terminal hair with a diameter of 30-120 $\mu$m is within the range of 0.6-10 ms. (See Altshuler G.B., Anderson R.R., Zenzie H.H., Smirnov M.Z.:; Extended Theory of Selective Photothermolysis, Lasers in Surgery and Medicine 29:416-432, 2001). Therefore, pulses with duration up to 10 ms are suitable and effective for the destruction of a hair matrix or the switching of the hair growth cycle due heating of the hair matrix. Hair papilla may be damaged by direct absorption of light in the micro vessels. However, a better way to damage the papilla of a follicle may be the diffusion of a thermal front at a temperature sufficient to damage tissue (~65°C - 75°C) from the hair matrix to the papilla. The time for this diffusion, which is sometimes referred to as the thermal damage time (TDT), is 15-20 ms for hair with the dimensions previously discussed. TDT of a whole follicle structure, i.e. the time of the propagation of the front of thermal tissue damage from the hair shaft or hair matrix to the outer junction of hair follicle, is approximately 30-2000 ms depending on the dimension of the follicle and on radiation intensity. In this case, the intensity should be limited in order to maintain absorption by melanin of hair shaft or hair matrix to the end of the pulse, (i.e., to prevent destruction of the hair shaft or hair matrix during the pulse).

For a hair shaft, this corresponds to heating the shaft to a temperature of less than 250°C. At the same time, the pulse should be long enough to deliver sufficient energy to the follicle for its destruction. Thus, the optimum pulse duration is TDT of the follicle structure as a whole. TDT of hair follicle (30-2000 ms) is essentially longer than the thermal relaxation time of the absorption layer in epidermis (320 ms). When long pulses with TDT duration are used, the temperature of the epidermis must be decreased by cooling so that much more energy may be applied to the follicle without risking damage to the epidermis. The effect of long pulse can not be simulated exactly by a train consisting of several short (up to 10 ms) pulses because the peak intensity of the short pulse may be high enough to destroy the chromophore in the hair follicle or to damage the epidermis. The temporal shape of the pulse is also important. Thus the shape of the pulse depends on the nature of the epidermis, dispersion of the hair diameters and length, hair shaft pigmentation and the cooling.

In Fig. 11, three main pulse shapes used for maximum hair follicle destruction are shown, the shapes being dependent on these three factors. These pulses will be referred to as the profiled pulses (PP). Curve 44 is the shape of a lamp pulse with front $\tau_f$ and trailing edge $\tau_r$ durations, where $\tau_f < \tau_r$. The duration $\tau_f$ should be considerably longer than the thermal relaxation time (TRT) of the epidermis, but much shorter than TDT of the target TRT<< $\tau_f$ <<TDT. The duration $\tau_r$ should be approximately equal to TDT. The heating mode provided by pulse type 44 allows rapid heating of the chromophore in the target (hair shaft or hair matrix) up to a maximum temperature where the chromophore is still not bleached and is viable and then maintains these temperatures (i.e. does not overheat the chromophore). The temperature of the chromophore (hair shaft or hair matrix) is thus kept nearly constant and close to the temperature of chromophore destruction. The pulse temperature has a substantially uniform shape.

For a pulse with shape 44 with rapid heating of the hair shaft or hair matrix up to maximum temperature, the efficiency of the absorption increases due to the denaturation of the surrounding tissues and scattering increase. Carbonization of chromophore and surrounding tissues may also take place causing an increase in absorption. If pre-cooling of the epidermis takes place, epidermal temperature and the temperature of surrounding tissue (including the contact cooler) is low and partially compensates for the heating effect by the front part of the pulse. Moreover as soon as $\tau_f$ << TRT during heating by the front part of the pulse, the epidermis is cooled due to the heat leakage into surrounding pre-cooled tissues. The decrease of power at the edge of the pulse protects the epidermis against overheating during the input of energy to the skin at the edge of the pulse. In this case, parallel cooling using the contact waveguide is especially effective.

Curve 45, a quasi-uniform pulse, has a pulse rise duration $\tau_f$ and a flat top of duration $\tau_m$. The power of the pulse on the top is selected in such way that $\tau_m \approx$ TDT is realized only near the end of the pulse and the temperature of the chromophore reaches maximum value just before the absorption of the chromophore decreases. This heating mode of curve 45 requires less power but longer TDT and higher total energy. The advantage of this mode is that it does not require as strong pre-cooling as the mode described by curve 44 and the output power of power supply 10 may be minimized.

Curve 46 describes a light pulse with long rise time $\tau_1$ and a short higher power end pulse with the duration $\tau_2$. Such pulse may be most effective for the treatment of patients who have high dispersion of pigmentation and hair diameters. In this case, follicles with strong absorption are initially damaged and at the end of the pulse the follicles with low absorption which need higher power are damaged. The light pulse with shape 46 may be effective due to the pre-heating effect of the front part of the pulse with the duration $\tau_1$. In this case, in the interval $\tau_1$ (0.1 - 5 s), the temperature of the lamp is low and it radiates much energy in the range of water absorption. Therefore, at this stage, pre-heating of the epidermis and hypodermis (where hair bulb is situated) takes place, and the temperature of the epidermis is kept low due to the parallel cooling by the contact waveguide 5. During stage $\tau_2$, which lasts approximately TDT, damage of the target takes place, while the temperature of the target is 45 - 60C and damage requires little energy. Functions describing the front and edge parts of light pulses 44, 45, 46 may be stair-like, linear, quadratic, exponential or other similar functions. In Table 1, the modes of hair management using the proposed device are represented. These modes are obtained based on numerical optimization taking into account the requirements of optimum energy utilization and desired cost.

<u>Vascular Lesion</u>

The described device is most effective for the treatment of vascular lesions with careful optimization of the filtered lamp spectrum, pulse duration and shape. For the treatment of shallow vascular lesions, the size of the beam is not too important. For the treatment of deep veins, requirements on beam size are the same as for hair management considered above. The criteria for spectral optimization are similar to the above. However the spectra of hemoglobin shown in Fig. 3 should be taken into account. For white skin, the PSL can include blue light that is very effectively absorbed by blood and needs lower energy than for the yellow spectrum. Using blue light makes the device more effective. The duration and the shape of the pulse are selected to cause thermal damage of the vessel's wall as soon as thermal necrosis of the endothelia takes place. The power of the pulse should be enough to keep the temperature of blood within the range 65-75°C for TDT but never exceed 100°C. The shape of the pulse is selected from the three shapes represented in Fig. 11. It may be formed in the same way as for hair management. The application of the selective epidermal cooling allows a lamp spectrum to be used which is wider in the short-wavelength range and provides higher efficiency of lamp energy. In Table 2 (superficial spider vein, rosacea, plexus, port-vine stain, gemanginoma, etc), 3 (deeper vein, feed vascular) and 4 (deep large leg vein), the modes of treatment of a vascular lesion situated at different depths using the described device are represented on the basis of numerical optimization. As shown in tables 2, 3, optimum PSL for vascular treatment can include one, two (Fig. 7b) or three bands.

Pigmented Lesion

The described device may be used for the treatment of different pigmented lesions. Pigmented lesions are usually situated at depths of 50 - 300 $\mu$m; therefore, the size of the beam is not essential. In the spectrum of the radiation, all components that could be absorbed by melanin, including UV radiation, may be present. The duration of the pulse should be less than the shortest times of TRT for a pigmented lesion or layer thickness where lamp radiation penetrates. Some pigmented lesion treatments require damaging layers of surrounding tissue. In this case, the duration of the pulse should be less than the TDT of all target. Cooling may be used to reduce the pain effect and decrease the risk of blistering. In Table 3, the modes of treatment of pigmented lesions using the described device are represented on the basis of numerical optimization. Highly pigmented and/or deep lesion can be treated with a redder spectrum. Lowly pigmented and/or superficial lesions can be treated with a spectrum which is more in the green or blue.

Similar parameters can be used for tattoo treatment, but the optimum PSL for this treatment is one or several bands of wavelength filtered from a lamp spectrum for which the ratio of temperature rise of the tattoo particles or drying tissue to temperature rise of the epidermis is more than 1.

Skin Rejuvenation

Limited damage of the skin may stimulate the replacement of the damaged tissues by new tissue and improve the cosmetic properties of the skin. The described device may be used for this purpose, damaging tissue and surrounding blood vessels in the papillary and reticular dermis, pigmented basal membrane and collagen in the dermis. In the first two cases, the modes of the treatment and the parameters of the device should be close to that described above for the treatment of vascular lesions and pigmented lesions. In order to provide damage to deeper layers of the dermis (100 - 500 $\mu$m), absorption of water in combination with cooling of the skin surface may be used. In this case, the color temperature of the lamp should be low and spectral filters should select spectral components which are highly absorbed by water (see PSL of Fig. 7c). In Table 6, the modes of skin rejuvenation due to damage of the dermis at a depth (100 - 500 $\mu$m) are represented on the basis of numerical optimization. For skin rejuvenation, the profiled pulses (PP) (Fig. 11) may be used. Thus PP of curve type 44 are optimum for the destruction of thin layers of the dermis. PP of curve type 45 is optimum for the destruction of the deeper layers. PP of curve type 46 may be used to combine damage of the dermis due to the absorption of water and destruction of blood vessels and dermis closely situated to the basal layer. In this case, the pulsed irradiation according to curve 44 may be combined with switching of the device output spectrum. On the long part of curve 44 with duration $\tau_1$, the power of the lamp is low and the spectrum is shifted to the range of water absorption. In the short part of the pulse $\tau_2$, the power is increased rapidly and the spectral maximum moves towards the visible or UF range. The duration $\tau_2$ may be shorter than TDT of thin vessels (0.1 - 10 ms) and thin layers of the dermis (1- 20 ms). In order to provide switching of the spectrum, an additional spectral filter with controlled transmission or nonlinear spectral filter with transmission spectrum dependent on the power of the lamp radiation may be used.

New collagen growth can also be achieved as the result of an inflammatory reaction around small blood vessels in papillary dermis. In this case, the treatment parameters are the same as in Table 2. This mode of treatment can be either in addition to or instead of the mode of achieving collagen growth previously discussed.

Acne Treatment

Acne vulgaris is one of the most common skin diseases and relates to hyperactivity of the sebaceous gland and acne bacteria. Lamp radiation may be used to reduce bacteria growth and for temporal or permanent damage of the sebaceous gland structure. In order to reduce bacteria growth, the photodynamic effect may be used on the porphyrins contributing to bacteria. Porphyrins have a modulated wide spectrum of absorption from red to the UV range. The optimum treatment mode is prolonged (1-30 min) irradiation of acne by lamp light in CW mode in the spectral range 340-1200 nm with the spectrum band(s) utilized being selected to match the absorption spectrum of the porphyrins. The intensity of the light delivered to bacteria (depth is 0-3 mm) should be as high as possible. In the proposed device, it is provided by intensive parallel cooling of the epidermis simultaneously with irradiation. Thus, due to the cooling (-5 - +5C), blood circulation in vessels of the papillary dermis is reduced and transmission of the skin dermis for blue and UV light is increased. Increased transmission may also be achieved due to pressure applied to the skin by waveguide 5.

According to the described method, it is possible to deliver to the skin lamp radiation with an intensity up to 20 $W/cm^2$ within the range 340-900 nm. Thus the short-wavelength part of the spectrum, for example 410 nm, is absorbed more intensively by propherin, but this absorption is reduced considerably at a depth - 0.5mm. At the same time, the red radiation is weakly absorbed by propherin, but is barely reduced at a depth 1 mm. Therefore, a wide spectrum is most effective to injure the bacteria via the photo dynamic effect.

The second and more effective mechanism of the treatment of acne vulgaris is reducing the sebum production function of the sebaceous gland. This may be achieved by the destruction of sebocytes or the coagulation of blood vessels supplying the sebocytes with nutrient substances. During periods of hyperactivity of sebocytes, the blood vessel net is filled by blood. The combination of a wide-band (340 - 2400 nm) light source with water filtering which attenuates radiation in the range of water absorption bands (1400 - 1900 nm) and with intensive cooling (-5 - +5C) of the epidermis and pressing of the skin, allows selective damage of spider veins supplying the sebaceous gland. Thus, the duration of the pulse should correlate with TDT of these vessels and may be about 1-100 ms for an energy density 5 - 50 $J/cm^2$, the energy density increasing with increasing pulse length. In order to totally or partially damage the sebaceous gland, it is possible to use a direct diffusion channel between the skin surface and the sebaceous gland. This channel is represented by the gap between the hair shaft and outer root sheath and usually is filled by sebum. Molecules and particles with dimensions less than $3\mu m$ with lyophil properties may diffuse through this gap and accumulate in the sebaceous gland. Further, these molecules and particles may be used for the selective photothermolisis of the sebaceous gland by lamp radiation. For this purpose, the lamp radiation spectrum has to be filtered so that its filtered part becomes the same as the absorption spectrum of the molecules and particles. For example: organic dye molecules, melanin, carbon, flueren with PDT effect, Au, Cu, Ag particle with plasma resonance can increase irradience around particles. The duration of the pulse should be shorter than the time of thermal relaxation of the sebaceous gland which is 50-1000 ms.

The intensity and fluence depend on the concentration and extinction of the molecules or particles but they should not exceed the threshold of epidermis damage or destruction. Therefore, cooling of the epidermis may be used to increase the efficiency of the destruction. For more effective delivery of the absorbing molecules and particles to the sebaceous gland, they may be combined with the lyophil particles. Dye molecules may be represented by the molecules of food dye, dye used for hair coloring and others. The particles may be represented by particles of melanin, carbon (for example, Indian ink), etc. Molecules of fulleren (for example, $C_{60}$) are among the most effective. These molecules have broad band absorption spectrum in the visible range. The important property of these molecules is the generation of singlet oxygen under photoexcitation. Singlet oxygen may additionally damage the sebocytes and bacteria. The insertion of the absorbing molecules and particles into the sebaceous gland may be done by heating of the skin, phonophoresis, electrophoresis magnetophoresis (if the particles have electric or magnet moment).

Particles inserted into a hair follicle and sebocytes may be used for hair management. In this case the contrast in absorption of the hair follicle with respect to the epidermis may be increased. This makes the treatment of light/gray hair and highly pigmented skin easier and provides more permanent hair loss (i.e. the absorbing particles or the molecules can be easily delivered into the region close to the bulge). The sebaceous gland may also be destroyed by utilizing the selectivity of specific heat of the gland vs. surrounding dermis, this selectivity being due to the high concentration of lipids in the gland. Thus, the gland may be heated by using band(s) of the spectrum with high water/lipid absorption and deep penetration, for example 0.85 - 1.85 $\mu m$ with cutting/filtering of the strong peak of absorption of water surround 1.4 $\mu m$ by a 1-3mm water filter and selective cooling of the dermis up to the depth of the sebaceous glands (0.5-1mm).

Based on the above, preferable components for the device D shown in Figs. 1, 2 are now considered.

Lamp

The lamp 2 in the device shown in Fig. 1 may be a gas discharge lamp based on the inertial gases Xe, Kr, Ne and others, a metal halide lamp, mercury vapor lamp, high pressure sodium lamp, fluorescent lamp, halogen lamp, incandescent lamp etc. The lamp has a linear tube shape. - Other variations include U shape or ring shape. The dimensions of the lamp are chosen on the basis of the device output parameters. For linear tubular lamps, the optimum shape of the output beam is rectangular a x b. The length of the discharge gap, that is distance 1 between electrodes, is chosen to be equal or bigger than one of the rectangular dimensions b. The inner diameter of the lamp should be minimized, but be sufficient to provide a given life-time N of the lamp (where N = number of lamp working cycles). Minimum lamp diameter provides the highest efficiency for transport of radiation energy to the skin and minimum losses of light due to absorption in the lamp. Minimum absorption of light inside the lamp increases the efficiency of back-reflected light from the skin. For low pulse repetition rate, the lamp may be cooled by the gas in gap 7, and for high repetition rate and high mean power, by a liquid in gap 7. The lamp tube may contain ions absorbing unwanted spectral components and converting these components into the desired spectral range. The optimum way to accomplish this is for the coating to reflect the unwanted radiation back into the lamp. This increases the efficiency of the lamp in the desired spectral range due to additional absorption of the reflected components in plasma.

Reflector

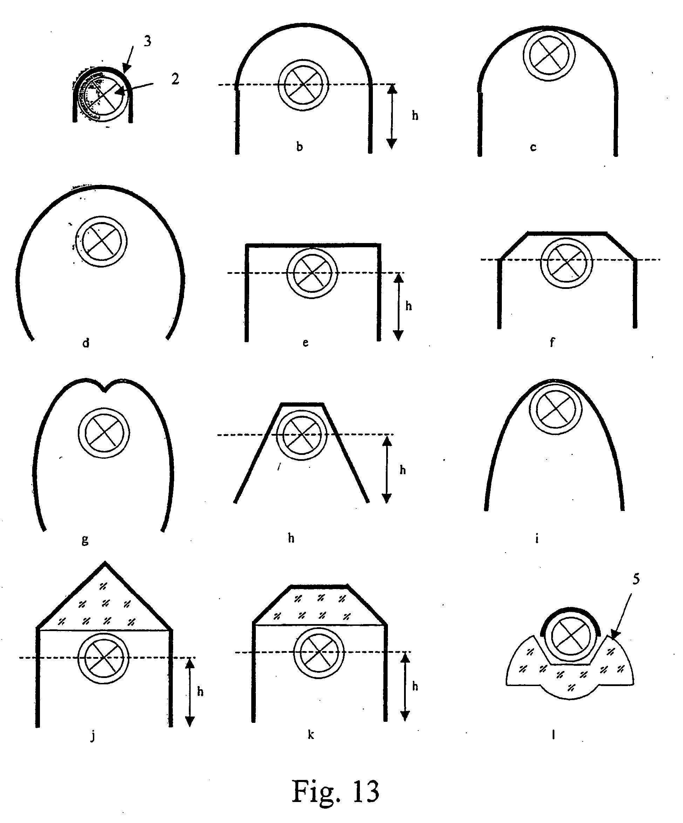

The reflector 3 may have various shapes (Fig.13). The main conditions providing maximum reflector efficiency are the following:

1. The ratio of the sum of the areas of the reflector's components providing significant reflection to the sum of the areas of the reflector's components which provide little or no reflection must be maximized. To provide this condition, the reflection index for working parts of the reflection must be close to one within the working range of spectrum. The best material for the specular reflector is Ag (visible or IR range) or Al (UV range). The reflector may be coated by a polymer or inorganic coating or the coating may be coated on the inside or outside of tube 4 or on lamp 2. In the later case, foil extending from the tube or other reflecting wings may extend to the waveguide to minimize photon loss. For a diffuse reflector, $BaSO_4$ powder may be used. The area of low-reflecting or non-reflection components in planes which are perpendicular to the axis of the lamp should be minimized. If this requirement is satisfied, the design of the device will become simpler and it will be possible to avoid cooling of the reflector.

2. The geometry of the specular reflector should provide the minimum number of reflections of lamp light from reflector 3 before being coupled into the waveguide. The reason for this is that there is a photon loss of about 5% to 15% per reflection; therefore, the lower the number of reflections, the less the photon losses. One way to reduce the number of reflections is to keep the reflector as small as possible, generally by moving the reflector close to the lamp. Under high color temperature of the lamp (T > 6000K), the total length of the path for the rays going across the lamp discharge gap should also be minimized in order to reduce losses due to absorption inside the lamp. A diffuse reflector has less efficiency than a specular reflector because the number of reflections from the lower reflective surfaces is greater than for the optimum specular reflector and the total length of the light paths inside the lamp is longer. However the diffuse reflector may have high efficiency if the area of low-reflecting components of the reflector is small and the lamp has low color temperature. For these conditions, angular spectrum at the output of the device will be widest. Therefore, this reflector may be used in cases which do not require deep penetration of light into the skin, for example, for skin rejuvenation and for pigmented lesions, but not for deep spider veins. The specular reflector for this device may be imaging or non-imaging. An imaging reflector is advantageous for the concentration of lamp light to a spot of minimum size, especially where the dimensions of the emitting source are small. However, where the dimensions of the emitting source are large, an imaging reflector is disadvantageous because the radiator is placed inside the handpiece. The cost of these reflectors is also high (i.e. they need far better quality reflector components).

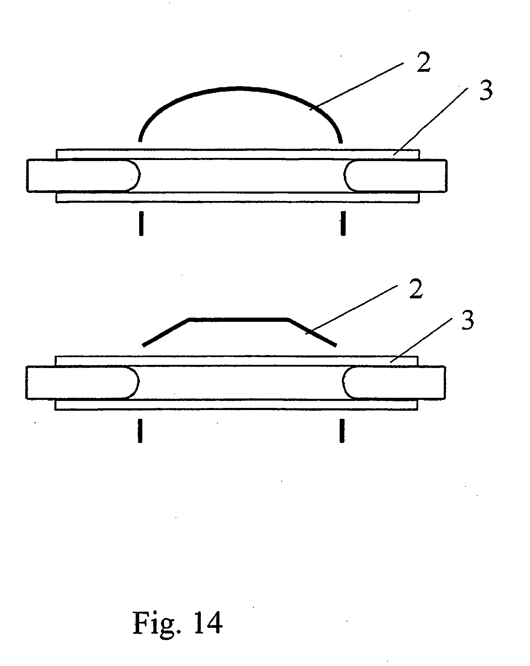

Non-imaging reflectors have lower efficiency; however, they are cheaper, have smaller dimensions and could provide more uniform irradiation for large spot size. In table 5, values of efficiency for the different specular reflectors shown in Fig.13 are represented. The dimensions of the lamp are 5x50 mm, the mean absorption in the lamp is 0.1 cm$^{-1}$ (Tc=6000K) and the reflection index of the reflector is 0.94. The distance between the center of the lamp 2 and the waveguide input is h = 7.5 mm (excluding reflectors shown in 13a, 13c, and 13ℓ. As can be seen from table 5, efficiency for the represented reflectors differ within a 12% range. An increase in efficiency of the reflectors may be achieved by reducing the number of lamp rays which impinge on the reflector surfaces where the electrodes and gaps for lamp cooling are situated. In order to provide this specification, the axial cross-section of the reflector (Fig.14) may be represented as a curved surface (sphere, parabola, ellipse) with its center situated in the center of the lamp or as a trapezoid. However, this increases the cost of construction. A construction which is both simple and effective is the reflector shown in fig. 13a or 13b. In this reflector, the reflecting surface has the shape of a simple cylinder and may be combined with the surface of the lamp envelope or tube 4. In the first case, cooling of the lamp and the reflector may be done outside the reflector, and in the second case, inside the tube. Further, since the electrodes are generally non-reflecting, they can be a major source of photon loss. One option is to use lamps without electrodes which are charged or excited by RF or other suitable techniques. Another option is to us electrodes formed of a material having high reflection.

Waveguide

The waveguide has the following functions in the described device:

1. The optical conjugation between the reflector 3 and the skin 1 (i.e. the transportation of lamp light and reflected light to the skin and back with minimum losses). In other words, an optical system with minimum photon leakage is provided and the waveguide is also a major factor in the increase in skin illumination resulting from the return or recycling of photons.

2. The creation of uniform illumination on the skin surface with fixed spot dimensions.

3. Cooling of the skin for the protection of the epidermis.

4. The pressing of the skin for the increased light transmission and better thermal and optical contact.

5. Laser or superluminescent conversion of the light.

6. Measurement of the index of light reflection from the skin in order to control the power of the light delivered into the skin depending on the properties of the skin.

7. Additional mechanical and electrical isolation of the skin from the lamp in order to increase patient safety.

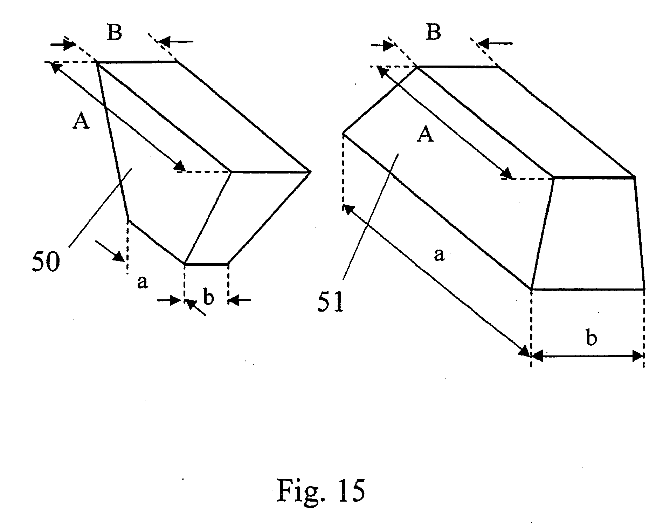

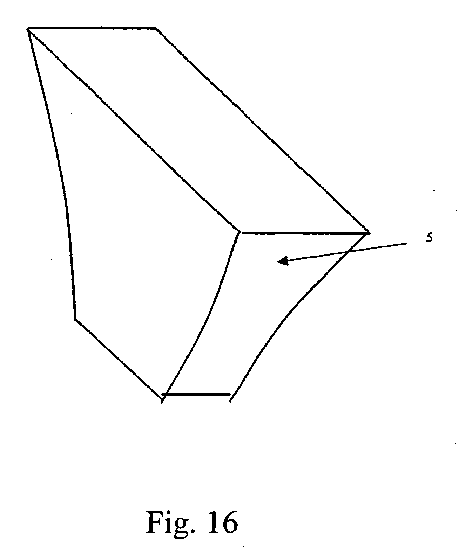

Waveguide 5 may be in the form of a rectangular prism (Fig.1), cut pyramid (Fig.15), or complex curvature cut pyramid (Fig.16). For a rectangular prism without coatings, the refraction index should satisfy the condition n>1.4, where n is the refractive index of the waveguide, for the transport of the radiation from the lamp to the skin without losses, and n>1.7 for the return of photons reflected from the skin back into the skin. Thus, an air gap should be provided between lamp 2 or tube 4 and waveguide 6. In order to provide uniform illumination on the skin surface and minimum photons loss, the gap between tube 4 and the waveguide should be of minimum size. While point contact between the lamp and waveguide may be possible, potential vibration of the lamp makes this a less desirable option.

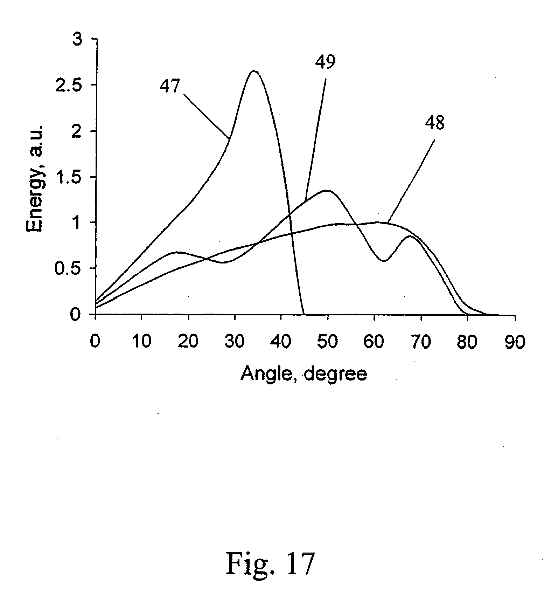

In Fig.17, the dependence of the non-uniformity of skin illumination on the length of the waveguide (the dimensions of the lamp 5x50 mm, the transverse dimension of the waveguide 16x46, the refraction index of the waveguide 1.76) is represented. Waveguide 5 may be in the form of a cut right-angle pyramid (Fig.15) or a curved pyramid (Fig. 16) prism for increased intensity of the fluence on the skin surface. The curved cut pyramid also allows transformation of the rectangular spot into a symmetric square or circle. The maximum value of the concentration of energy density is achieved if losses in the waveguide are not high and the ratio of the square of the input aperture to the output aperture is maximum.

If the losses in the waveguide are limited to 5%, the maximum concentration (i.e. the ratio of energy density on the skin surface with the cut-off pyramid (Fig.15) to the energy density on the skin surface with the right-angle prism (Fig.1) will be achieved for certain angles of the pyramid defined in two dimensions. For the long axis, this angle is equal to 17°, and for the short axis, is equal to 3.8.

The length of the waveguide is limited by absorption losses of the waveguide and by the dimensions of the handpiece. For a waveguide length H=60 mm A=46 mm, B=16 mm; the maximum concentration of light by a cut-off pyramid in comparison with a right-angle prism is equal to 1.95 for $n_w$=1.45(quartz) and 2.3 for $n_w$=1.76 (sapphire). A equals the length of the waveguide along the long axis at the light receiving end of the waveguide, and B equals the length along the short axis.

The width of the angular spectrum coupled into the skin by the waveguide depends on the refraction index of the medium placed in the gap between the tube 4 and the waveguide as well as on the angle of the pyramid. In Fig.17, the angular radiation spectra from the device (Fig.1) in the skin near the surface (ballistic photons) are represented. Curve 47 shows the angular energy distribution of the ballistic photons in the skin for the device (Fig. 1,2) with a sapphire waveguide made as a right-angle prism (A=46 mm, B=16 mm, H=15 mm) and air in the gap between tube 4 and waveguide 5. Curve 48 describes the same situation; however the gap between the tube 4 and waveguide 5 is filled with a transparent substance with a refraction index equal to n=1.42. Curve 49 describes the angular distribution of the energy of ballistic photons for the waveguide made as a cut-off quartz pyramid (A=46 mm, B=16 mm, a=11.6 mm, b=28 mm, H=50 mm). From Fig.17, it is seen that it is possible to control the angular spectrum of the photons inside the skin using waveguide 5 and changing the refraction index of the substance placed between the tube and the waveguide. In accordance with well-known theory, changing the angular spectrum of the photons inside the skin is the best way to control the depth of penetration of light into the skin, especially for long waves. In order to achieve an extremely narrow angular spectrum and maximum penetration depth, air should fill the gap between tube 4 and waveguide 5 and the waveguide should be made as a right-angle prism or as "divergent" cut-off pyramid 51 (Fig.15). The surface AxB is faced to the lamp and axb is in contact with the skin. This shape is most suitable for the treatment of deep targets such as hair bulge, hair bulb, dermal/ hypodermal junction, subcutaneous fat, deep veins, etc. In order to provide maximum angular spectrum and minimum depth of light penetration into the skin, the space between the tube and the waveguide should be filled with a substance with a refraction index greater than 1, preferably equal to or greater than the refraction index of the skin, but less than the refraction index of the waveguide. The angular spectrum may be expanded additionally due to application of the waveguide made as a convergent cut-off pyramid 50. A device with high divergence of the radiation in the skin and low penetration depth may be used for pigmented lesions, vascular lesions and skin rejuvenation.

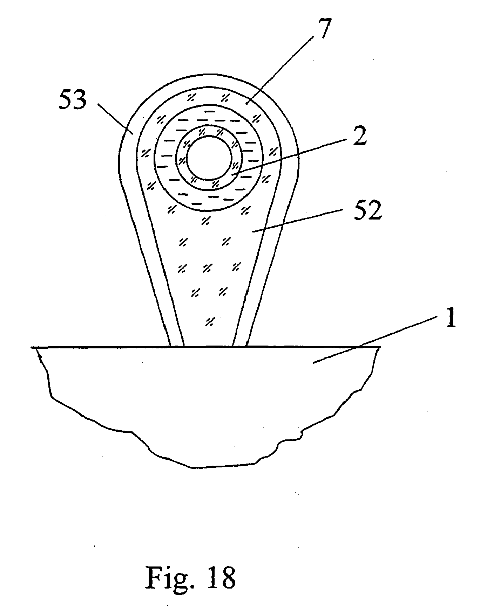

Fig.18 shows a device with the simplest waveguide combined with a reflecting tube providing maximum concentration of energy near the surface of the skin. In this device, waveguide 52 transforms smoothly to perform the function of tube 4, gap 7 being formed between this waveguide and the lamp. Reflector 53 is mounted on, coated on or otherwise formed on the waveguide. A reflector on the surface of waveguide 52 is necessary. In this embodiment, it is impossible to provide total internal reflection on the waveguide junction due to the wide angular spectrum of the radiation. Reflector 53 may be made as a vacuum or galvanic metal coating (Ag, Cu, Au, Al) on the dielectric waveguide 52 or as a flexible sheet with a reflecting coating. The flow of liquid or gas in gap 7 between the waveguide and the lamp is used for cooling both the waveguide 52 and the lamp 2 (and through the waveguide reflector 53).

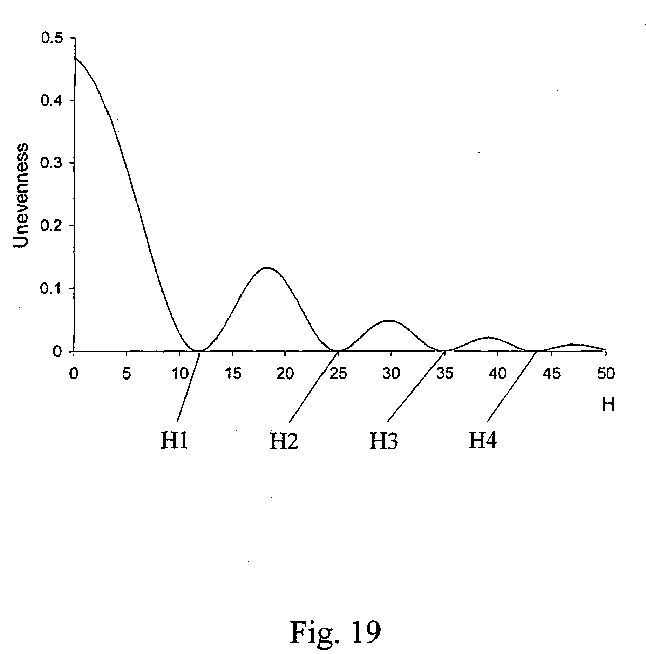

An important function of the waveguide is providing uniform distribution of radiation on the skin surface this being a critical parameter for the safety of the epidermis. Uniformity of illumination is provided due to the correct choice of waveguide's length. A typical dependence of radiation distribution intensity non-uniformity on skin surface 54 on the length H of the waveguide is shown in Fig.19. The non-uniformity (unevenness) Z is defined as $Z=(Imax-Imin)/2(Imax+Imin)$, where Imax is maximum and Imin is minimum energy density (power) on the skin surface. For better safety, $Z=0$. From Fig.19, it can be seen that this dependence has a periodic, resonant decreasing character for increasing H. For short waveguides when their length $H≈B$, the length of the waveguide should be close to the lengths for resonance H1, H2, H3, H4. For $H»B$, the radiation distribution is uniform independent of the length H of the waveguide.

In order to provide maximum coupling efficiency of lamp radiation into the skin, the front face of waveguide 52 should be in optical contact with skin 1. To provide this, the waveguide is pressed against the skin and all gaps between the waveguide's output plane and skin more than $0.2\mu m$ should be filled with a liquid with a refraction index $n>1.2$. In order to minimize these gaps, it is useful to expand the skin in the contact field. Good optical contact automatically provides good thermal contact between waveguide 5 and skin 1. The pressing of the skin by the waveguide, especially in places near the bone or where there is a hard plate under the skin being treated, for example where there is a hard reflecting plate inserted in the gap between the inner lip and teeth/gum of the patient to prevent absorbtion of radiation by the patients teeth or fillings therein, and thus heating of the teeth where the patient's lip is being treated, allows considerable increase in the depth of light penetration into the skin. This effect is achieved due to decreased scattering in the skin under pressure and the removal of blood from underlying vessels. While what has been described above is clearly preferable, there may be applications where adequate optical contact can be obtained with the waveguide very close to, but not necessarily in contact with the skin.

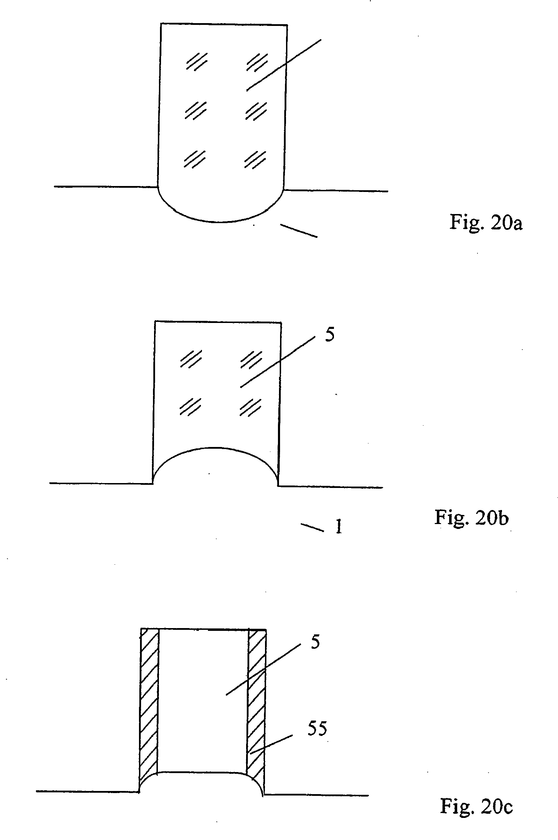

In order to increase pressure on the skin, the front face of the waveguide may be made in the form of a convex surface (Fig.20a). Where treatment of blood vessels is being performed, pressing of the skin should generally be avoided since blood in the vessel is generally the chromophore used for treatment. In this case the face of the waveguide may be made in the form of a concave surface (Fig.20b) or it may have a rim 55 (Fig.20c). Rim 55 or the sharp edges of the waveguide (Fig.20b) can block blood flow in the vessel on either side of the treatment field, resulting in a concentration of non-flowing blood in the treatment field.

The waveguides of, for example Figs.20b and 20c, may also be utilized to control the blood vessel being treated. In particular, there is a concentration of thin, for example $10\text{-}30\mu m$ blood vessels in the plexus which is located just below the dermis epidermis (DE) junction of the skin; below these plexus vessels are thicker, but still relatively thin, spider veins, and below the spider veins are thicker blood vessels. Generally treatment of the plexus vessels is not desired. However, radiation absorption in these vessels can both cause undesired heating of the plexus which then cause blistering and pain, and also absorbs energy, reducing the photons reaching the vessel on which treatment is desired. It is therefore desirable that these plexus vessels be compressed (and/or cool plexus) so as to remove blood therefrom, while not compressing the vessel to be treated. The recess of the waveguide of Fig. 20b or rim 55 (Fig. 20c) can be selected so that the top of the recess presses on the plexus vessels removing blood therefrom, while the edges of the recess only pinch the vessels on which treatment is to be preformed, trapping blood therein. A deeper recess in the waveguide/rim would permit blood to, for example, also be removed from spider veins to facilitate treatment of deeper, larger vessels. Thus, by controlling both the depth of the recess in the waveguide/rim and the pressure applied, the depth of the blood vessel being treated may be controlled. Red or blue light, depending on the vessel being treated, may be utilized to detect blood flow in vessels, and thus to provide feedback for controlling the pressure applied by the waveguide to the patient's skin. With the convex waveguide of Fig. 20a, control of pressure alone can be used to control the depth of the blood vessel being treated. This control of the depth of blood vessels being treated by use of a suitably shaped waveguide is another feature of the invention.

Skin texture improvement may also be achieved by the heating of small vessels in the plexus and superficial papillary dermis to produce an inflammatory reaction in the vessels, resulting in the production of elastin and stimulating fibroblast to grow new collagen. In this case, controlled compression of skin surrounding the treatment zone by rim 55 (Fig. 20c) can significantly increase vasculization of small vessels and increase efficiency of the treatment.

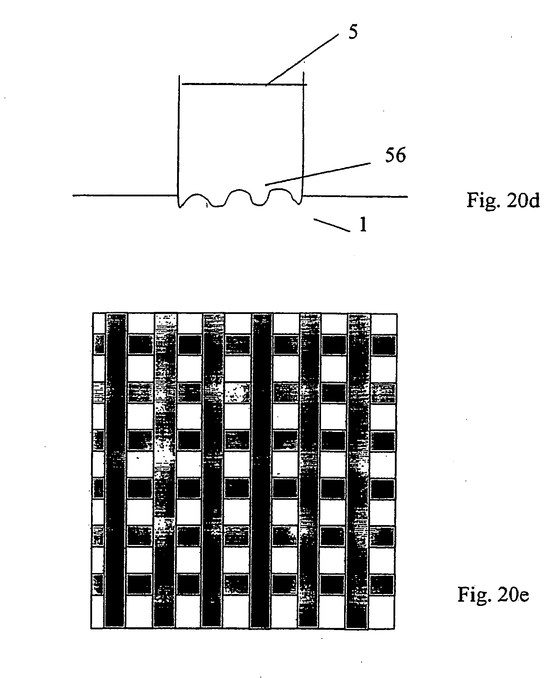

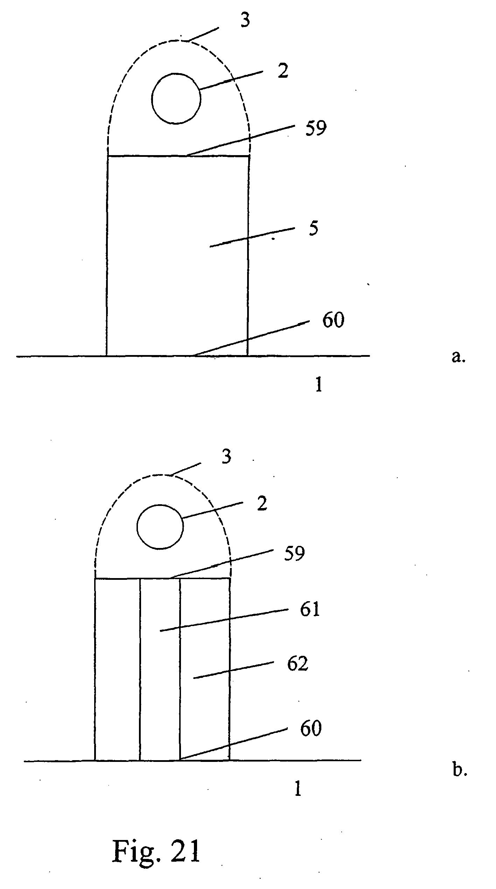

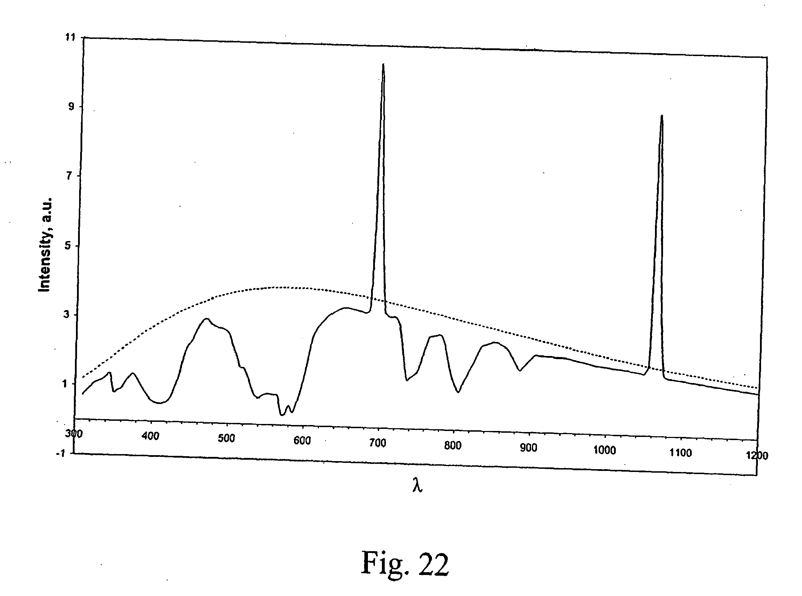

The output edge or face of the waveguide may have spatial non-uniformities. In this case, damage of the skin will be non-uniform. The size of the non-uniform fields may be less than $50\mu m$. The non-uniform damage may be useful for skin rejuvenation, or for vascular or pigmented lesions, tattoos, etc., because it decreases the peak of extremely strong damage of the skin: blistering, purpura etc. At the same time, the damaged islands heal quickly because tissue between the damaged islands is not damaged and can therefore provide cell proliferation. In order to provide non-uniform damage of the skin surface, the face of the waveguide may have a modulated profile 56 as is shown in Fig.20d. A spatial mask 58 (Fig.20e) may also be coated (reflected mask) on the front surface of the waveguide, for example a flat mask. Patterned index variations (phase mask) in the waveguide may also be employed. Other optical techniques may also be utilized to accomplish this objective. At least some of the techniques indicated redistribute light to provide selected treatment spots. Waveguide 5 may be made as a lasing or superluminescent waveguide. In this case, the wave spectrum of the lamp may be actively profiled and the angular spectrum of the lamp may be narrowed in order to provide delivery of the light to greater depths. Waveguide 5 may be partially or entirely made of a material impregnated by ions, atoms or molecules having absorption bands in the range of the lamp radiation and lasing or superluminescence transitions in the desired spectral range. Waveguide surfaces 59 and 60 (Fig. 21a) should be parallel with a high accuracy that provides minimum losses of laser generation (better than 30 minutes, preferably better than 10 seconds) and having a curvature which minimizes diffraction losses. Surfaces 59 and 60 have coatings, the coating on surface 59 having a refraction index which is close to 100% for lasing or superluminescent wavelengths and minimum refraction index for lamp radiation in the desired spectral range and within the range of the ions, atoms and molecules absorption. The coating on surface 60 has a refraction index of a value which is optimum for laser generation. In order to increase the intensity or fluence of laser generation, waveguide 5 may be made in two parts: active part 61 and passive part 62 (Fig. 21b). Active part 61 is doped and part 62 has no absorptive dopants. The waveguide may consist of several parts 61 and 62 or active parts 61 may be formed by spatially selective doping. High-reflecting coatings 59 and 60 may be made only on the edges of the active part of the waveguide. Additionally, the refraction index of the active part of the waveguide may be greater than the refraction index of the passive part in order to realize the waveguiding effect for laser radiation. The radiation of the lamp propagates along waveguide 5, intersects many times with active parts 61 and excites the active dopants. If the waveguide consists of several parts, the generation takes place in the elements 61 which have less cross-section than the waveguide. Therefore the radiation decreases wave and spatial spectra and increases the fluence. Suitable lasing materials include: $Cr^{3+}$:$Al^2O^3$, $Ti^{3+}$: $Al^2O^3$, Nd:YAG, SiO2:Rodamin 6G and others. Thus, the embodiment of Fig. 21b provides treatment with the combination of both a lamp and a laser, the waveguide 61 being a laser which is pumped by lamp 62; the combination is required since if the whole waveguide were formed from a laser, there would not be enough fluence for desired treatment, or in other words, there would not be enough gain. Fig.22 shows the radiation spectrum 63 of the proposed device. In this example, an active waveguide with the elements 61 made of ruby and Nd: YAG is used. This waveguide has coatings 59, 60 providing lasing at wavelengths of 694 nm and 1064 nm. The spectrum 64 of the lamp without waveguide is presented for comparison. Spectrum 63 may be efficient for the treatment of the deep veins.

Filtration of light

Optimum profiled spectrum of the lamp (OPSL) is determined by the treatment target. Optimum conditions are: 1) Temperature of epidermis is lower than temperature of thermal necrosis, 2) Temperature of the target is higher than temperature of thermal necrosis, 3) Loss of light energy in the filter is minimized. Mathematically it has been demonstrated that OPSL requires a sharp cutoff. Fig. 7a-7c show OPSL as a result of calculations following the above conditions: Fig. 7a being for mulatto skin/hair removal, Fig.7b being for white skin/spider vein treatment, and Fig.7c being for skin rejuvenation through collagen heating. Simple criteria for OPSL can involve one or more wavelength bands selected/ filtered from a lamp spectrum, the band(s) being selected such that the ratio of temperature rise of the target (hair shaft, matrix, vessel, vein, pigment lesion, tattoo, etc.) to temperature rise of the epidermis is more than certain numbers S. The number S depends on the desired level of safety for the procedure. Higher S gives a higher safety level. To maximize efficiency of the lamp, S should be about 1.

Filtration of the light spectrum can be realized by all the optical components of the proposed apparatus. Possible filtration mechanisms include wavelength selective absorption of light in lamp 2, the liquid in gap 7, tube 4, waveguide 5, filter 6, and the wavelength selective reflection of light at reflector 3. Filter 6 may be implemented as a multilayered dielectric coating, reflecting coating, absorbing medium, or spectral resonant scatterer.

Use of a reflecting coating as a filter is desirable to avoid additional losses of light, excess light heating, and to minimize required cooling. A filter of this kind augments the radiation efficiency of the lamp in the proposed device by the reabsorption of superfluous light in the lamp and the increasing of its light output. However, at large angles of incidence, a dielectric interference filter better transmits the short-wavelength part of the light spectrum to the skin than the long-wavelength part. This leads to additional heating of the epidermis useful for treatment of pigmented and vascular lesions only, provided the vascular lesions are very superficial. Conversely, an absorbing filter better transmits the long-wavelength part of the spectrum than the short-wavelength portion. This is better for the treatment of deeper targets and is safer for the epidermis. Unfortunately, an absorbing filter is heated by light and needs cooling. Therefore, it is most efficient to place this filter on lamp 2 or inside tube 4. If this is the case, liquid or gas in gap 7 cools the filter simultaneously with the lamp, the latter being the major source of heat. The filter may be implemented as absorbing dopes (ions, atoms, molecules, microcrystals) added to the liquid in gap 7 or to the material which lamp 2 or tube 4 is made of. Where water filtering is desired, the fluid in gap 7 may be water, either alone or doped as desired. Other fluids, such as oil, alcohol, etc. could also be usin in gap 7.

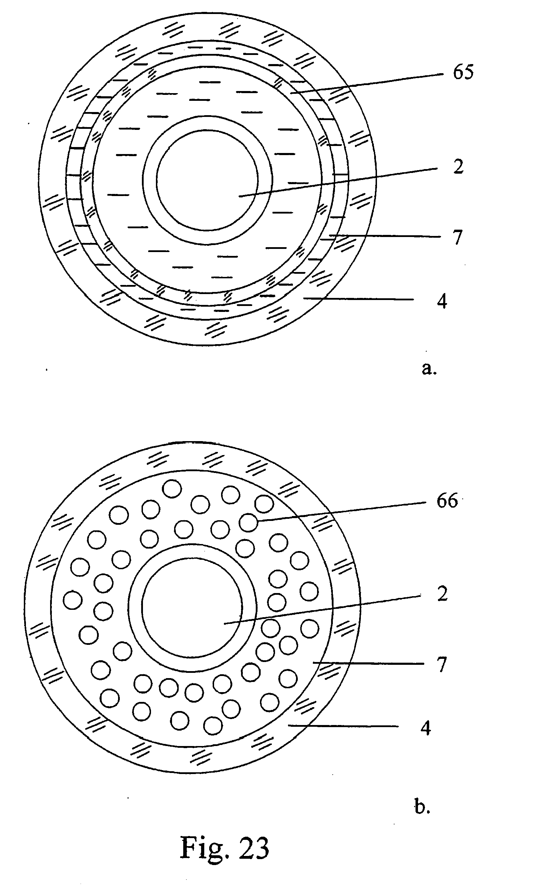

Moreover, an additional tube 65 (Fig. 23a) may be included inside tube 4, the former being made of absorbing material, for instance glass doped by Ce, Sm, Eu, Cr, Nd, La, Fe, Mg, Tm, Ho, Er, etc, ions or by semiconductor microcrystals. The tube may be replaced by particles or slabs, fibers or other components 66 of the same material (Fig. 23b) embedded into the cavity between lamp 2 and tube 4. Tube 65 and components 66 are cooled, the latter being an advantage because of the strong filtration and high average power of the apparatus proposed. The filtration may be implemented by using resonant scattering with respect to the indices of refraction. For instance, let the refraction index of particles 66 be chosen to coincide with that of the cooling liquid at wavelength $\lambda$. Then, there exists no scattering in the tube at wavelength and, therefore, the transmission is a maximum. As the wavelength is detuned from $\lambda$, the mismatch of refraction indices grows, reinforcing both the scattering and extinction of light. If the refractive index of at least one of the components 7 or 66 changes as a function of the power of the light or of temperature this scattering medium can automatically (self) regulate fluence on the tissue. For example, for low power, the difference in refractive indexes $\Delta n$ between 7 and 66 is minimum and attenuation of the light due to scattering is also minimum. But for high power, due to the non linearity of refractive indexes of 7 or (and) 66, $\Delta n$ increases and attenuation of light increases too. This mechanism can be used for protection of skin from high fluences. Filter 6 may be implemented using the same principle. In this case, the spectrum of transmittance may be controlled, for instance by an electric field, provided one of the scattering components exhibits a strong dependence on an electric field, for example liquid crystal or segnetelectrical ceramics The filter 6 can be made as a suspension of liquid (water as example) and solid state particles with matching refractive indexes $\Delta n \approx 0$ when the liquid is frozen (ice). Scattering and attenuation of light in this condition is very low. The temperature of waveguide 5 (around 0°C) will remain as melting temperature of filter 6 until the liquid is completely melted. This period of time can be used for treatment of skin with good cooling. Refractive indexes of medium in liquid and crystal conditions are very different. So, after melting, the liquid 6 is going be a high scattering plate with significant attenuation of the beam. When 6 loses its cooling capability, the fluence on the tissue will thus automatically drop to prevent tissue from damage.

To filter the light spectrum near the IR absorption peaks of water at 1.4 and 1.9 $\mu$m, a liquid water filter with a thickness of 1 - 3 mm may be used, which water may also be used for cooling.

<u>Cooling</u>

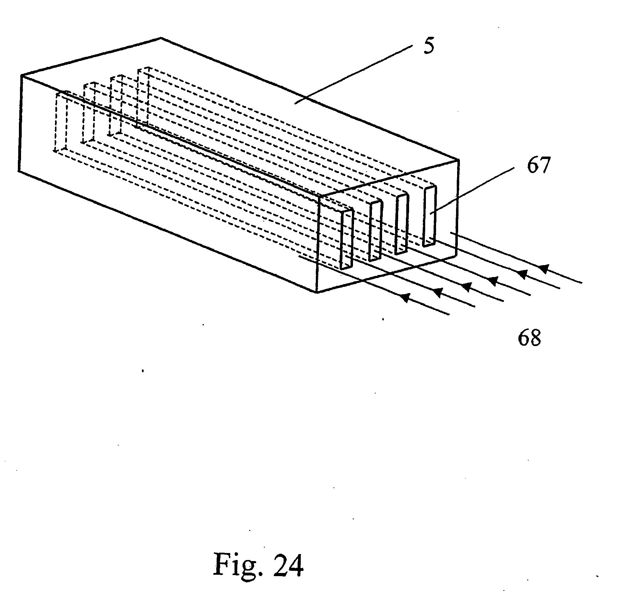

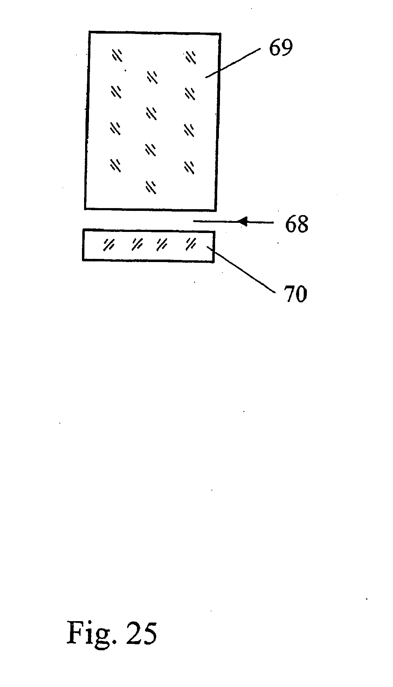

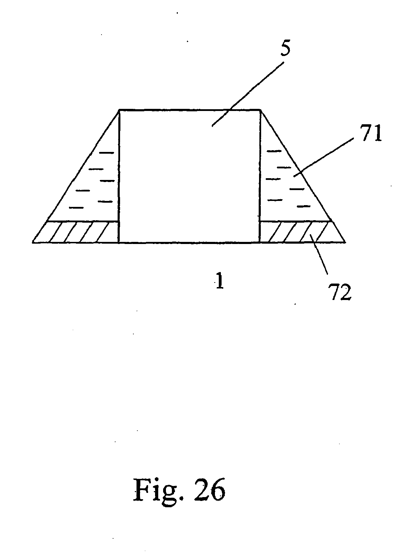

To increase the light energy deposited to the skin, the skin, may be selectively cooled. Cooling of skin to temperatures below 4 °C may be effective for reducing or eliminating pain. In the apparatus proposed, skin cooling is implemented through contact with the cooled tip of waveguide 5. Several mechanisms for cooling waveguide 5 are possible. Fig. 24 shows a cooling mechanism for waveguide 5 which is most effective for large A and B dimensions and significant heat flux from the skin (highly pigmented skin, long pulses). The waveguide of a material having good thermal conduction properties, such as sapphire, has a plurality of cuts 67 formed therethrough, with cooling liquid or gas circulating through the cuts. The cuts may have circular, rectangular or other cross-section. The inside surface of the cuts should exceed in total area that of the waveguide tip contacting the skin. The cuts are distributed uniformly over the waveguide, thereby eliminating temperature gradients or at least decreasing the gradients from what they would be if only the sides are cooled. The cooling may also be accomplished through evaporation of a liquid like freon from the cut surfaces. Fig. 25 shows a cooling mechanism in a composite waveguide assembled of a part 69 which may be of a poor heat-conducting material and a plate 70 of a highly heat-conducting material, cooling liquid or gas 68 circulating in and filling the thin gap between them. Furthermore, light- volatile liquid (for example evaporating spray as R134A) may be injected into the gap between 69 and 70. The mechanism of Fig. 25 also provides uniform cooling of skin for a large waveguide. Fig. 26 shows a cooling mechanism for the side surface of the waveguide, making use of circulating fluid, gas, or spray. The mechanism includes components 71 removing heat from the side surface of waveguide 5. Component 71 may be circulating cooling fluid or may be a Peltier or other thermoelectric component. This mechanism is applicable provided at least one dimension A, B is small enough. Additional plates 72 cooled by the same cooling components 71 may be provided, plates 72 being used to pre- and postcool the skin when the apparatus is scanned over the skin surface.

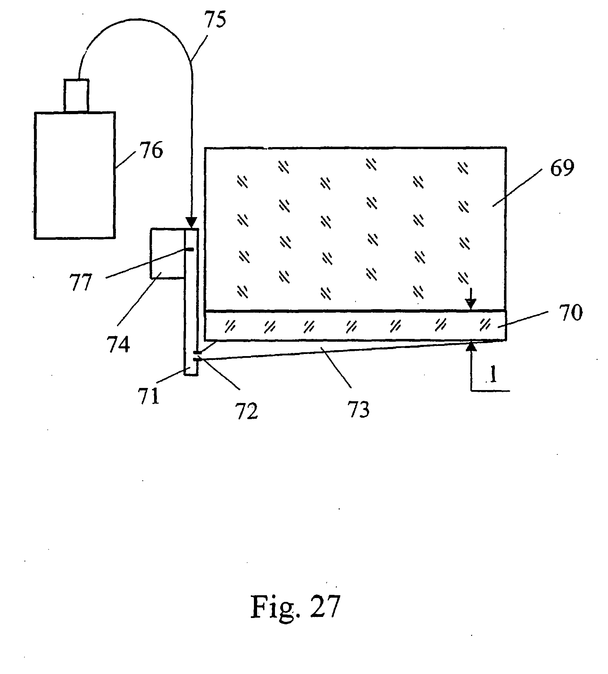

Fig. 27 shows composite waveguide 69, 70 cooled by a spray 73 of a fluid with a low evaporation temperature like freon. Reservoir 76 containing the liquefied fluid is connected through tube 75 to a valve 77 controlled by an electrical or mechanical mechanism 74. When valve 77 is opened, the liquefied gas is piped under pressure from reservoir 76 to tube 71 and is then sprayed through nozzle 72. The pulse duration while the valve is open is chosen to pipe enough fluid to component 70 to cool it to the prescribed temperature. This temperature, and the thickness of element 70, are chosen to cool the skin to the prescribed depth, preventing epidermal injury. Tube 71 preferably includes a contact sensor so that valve 77 is operated when tube 71 contacts the skin. It is seen that this occurs before element or plate 70 contacts the skin. This results in the cryogen or other cooling spray being applied both to the skin and to plate 70, resulting in a precooling of the skin and, when plate 70 comes into contact with the skin also in parallel cooling. The thickness of plate 70 can control the depth of cooling Component 70 may be made of sapphire or diamond; the material of waveguide 69 has to be heat insulated in part from waveguide 70 through at least one of its low heat conductivity and low heat capacity (for instance, plexiglass or glass) or by means of glue.

The advantage of the mechanism of Fig. 27 is that it prevents the overcooling of the epidermis for properly chosen thickness of plate 70 even though the initial temperature of plate 70 is low. Furthermore, the unavoidable (when not using sprays) temperature gradients smooth out when the fluid is sprayed onto plate 70. The fluid is sprayed before waveguide 70 touches the skin. Plate or waveguide 70 may be placed very close to the skin surface and, therefore, the sprayed fluid precools the waveguide and the skin simultaneously. Then, both optical and thermal contact between the skin and the waveguide are established, an optional time delay is introduced, and light from the lamp then irradiates the skin. Numeric simulations show that freon boiling at temperature -26° C cools the epidermis effectively, provided the sapphire plate thickness 1 is 0.5 - 3 mm. The precooling duration is 0.2 - 1 s. For all the processes to be synchronized, the mechanism of opening valve 77 is preferably controlled from a skin touching sensor, for example a sensor in tube 71.

For optical dematology apparatus where a cooling fluid, for example water or air, is flowed over a contact plate 70, the thickness of this plate may also be selected to control the depth of cooling as for the plate 70 of Fig. 27.

Additional Safety Measures

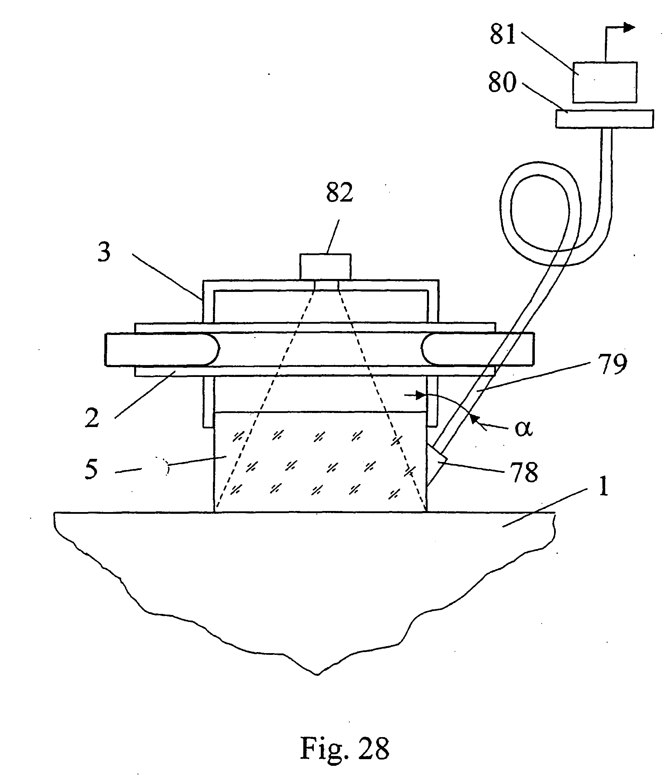

The device of this invention is not only intended for using by a physician, but also for salons, barber shops and possibly home use. For this above reason, one version is supplied with a system for detecting contact with the skin. The system prevents light irradiation of the human's eye and may also evaluate the pigmentation of a patient's skin. The latter capability, in particular, provides a capability to automatically determine the safest irradiation parameters for a particular patient. An embodiment of such detection system is shown in Fig. 28. Light from arc lamp 2 or additional light source 82 (microlamp, waveguide) is directed to the outlet of waveguide 5. Optical fiber 79 is coupled to waveguide 5 by for instance prism 78. Angle $\alpha$ is chosen to minimize or prevent light from lamp 2 or light source 82 from passing through prism 78 so that ideally only light (photons) reflected from skin 1 reach detector 81. Ranges for the angle $\alpha$ fall within the following limits: $\arcsin\left(\dfrac{1}{n_W}\right) < \alpha < 90°$. For sapphire 34.6°<$\alpha$<90°. On touching the skin, backscattered light from the skin enters waveguide 78. Within the waveguide, the backscattered light has a broader angle spectrum than the direct light from 2 or 82. The former light propagates within the angle range $\dfrac{n_{skin}}{n_W} < \alpha < 90°$. For sapphire this yields 53.8°<$\alpha$<90°. Therefore, if the condition $\left(\dfrac{1}{n_W}\right) < \alpha < \arcsin\left(\dfrac{n_{skin}}{n_W}\right)$ holds, and the angular aperture of the waveguide is within this angle range, then no light other than backscattered light from the skin enters waveguide 78. The intensity of this light depends on the skin type, especially within a preferable spectral range 600nm<$\lambda$<800nm. The reflected signal is measured by photodetector 81 through filter 80 which cuts off undesirable wavelengths. The output from photodetector 81 is utilized by the system to control power supply 10 (Fig. 2). The minimal signal level reached for perfect optical contact of the waveguide with the skin is preset based on the diffuse reflection coefficient for the patient skin type. Contact detection is facilitated by the fact that the signal applied to detector 81 jumps significantly on contact. Filter 80 assures this occurs only for the reflected light. The optical system of Fig. 27 protects the skin from injury caused by variations in skin parameters, for instance by inhomogeneous pigmentation. Photodetector 81 may be connected directly to waveguide 5. Moreover, the apparatus is also capable of being controlled based on measurements of the irradiance inside the optical system undergoing minimal photon leakage. This irradiance is proportional to the output energy of the lamp if the lamp is emitting in air or to a standard reflector. But this irradiance proportional to the reflection from the skin if the lamp is emitting in skin. In the latter case, the optical system works like an integrating sphere.

While the invention has been described above with respect to multiple embodiments, and many variations have been discussed, these descriptions are for purposes of illustration only, and further variations may be made therein by ones skilled in the art while still remaining within the spirit and scope of the invention which is to be defined only by the appended claims. For example, while the concepts discussed above have been used in a lamp based implementation, many of these concepts are not limited to use only in a system using a lamp as the radiation source, or even to the use of a non-coherent radiation source

All wavelengths in the following tables are determined with tolerance +/-5%. For example, 0.51μm means 0.485-0.536μm

Table 1.

Characteristics of flashlamp radiation for hair removal

| Skin type | Color Temperature of lamp, °K | Light spectrum | | Pulsewidth ms | Fluence, $J/cm^2$ (Treatment of bulb, after filtering) | Fluence, $J/cm^2$ (Treatment of bulge, after filtering) | Beam width, mm (Treatment of bulb) | Beam width, mm (Treatment of bulge) | Cooling temperature, °C |
|---|---|---|---|---|---|---|---|---|---|
| | | Thickness of water filter, mm | Short cut off wavelengths, μm | | | | | | |
| I-II | 4000-7000 | 0-5 | 0.51-0.6 | 1-1000 | 1-40 | 5-100 | >15 | >8 | -5-36 (0.1-1 sec) |
| III-IV | 3000-6000 | 0-5 | 0.5-0.7 | 1-1000 | 1-40 | 5-100 | >15 | >8 | -5-36 (0.1-2 sec) |
| V-VI | 3000-5000 | 0-5 | 0.6-0.8 | 1-1000 | 1-20 | 5-50 | >15 | >8 | -5-30 (1-2 sec) |

EP 1 665 996 A2

Table 2.

Characteristics of flashlamp radiation for small superficial vascular treatment, and treatment of superficial blood vessels for texture/wrinkle improvement

| Skin type | Color Temperature of lamp, °K | Light spectrum | | Pulsewidth ms | Fluence, J/cm² (after filtering) | Beam width, mm | Cooling temperature, °C |
|---|---|---|---|---|---|---|---|
| | | Thickness of water filter, mm | Bands of wavelengths, μm | | | | |
| I-IV | 5000-10000 | 0-3 | 1.  0.38-0.47<br>2.  0.38-0.6<br>3.  0.38-047 & 0.51-0.6<br>4.  0.51-0.6<br>5.  0.38-0.6 & 0.75-1.3 | 0.1-50 | 0.5-50 | >3 or island spots | -5-36 (0.1-0.3 sec) |
| V-VI | 5000-7000 | 0-3 | 1.  0.51-0.6<br>2.  0.51-0.6 & 0.75-1.3<br>3.  0.75-1.3 | 0.1-100 | 0.4-20 | >3 or island spots | -5-10 (0.1-0.3 sec) |

Table 3.

Characteristics of flashlamp radiation deep vein treatment (0.2-0.5mm; depth 0.5-1mm)

| Skin type | Color Temperature of lamp, °K | Light spectrum | | Pulsewidth ms | Fluence, J/cm$^2$ (after filtering) | Beam width, mm | Cooling temperature, °C |
| --- | --- | --- | --- | --- | --- | --- | --- |
| | | Thickness of water filter, mm | Bands of wavelengths, μm | | | | |
| I-IV | 4000-10000 | 0-3 | 1. 0.51-0.6<br>2. 0.51-0.6 & 0.75-1.3 | 5-1000 | 2-100 | >5 or island spots | -5-36 (0.1-1 sec) |
| V-VI | 3000-6000 | 0-3 | 1. 0.51-0.6 & 0.75-1.3<br>2. 0.75-1.3 | 5-2000. | 10-100 | >5 or island spots | -5-20 (1-2 sec) |

EP 1 665 996 A2

Table 4.

Characteristics of flashlamp radiation for large vein leg treatment

| Skin type | Color temperature of lamp, °K | Light spectrum | | Pulsewidth ms | Fluence, $J/cm^2$ (after filtering) | Beam width, mm | Cooling temperature, °C |
| | | Thickness of water filter, mm | Bands of wavelengths, μm | | | | |
|---|---|---|---|---|---|---|---|
| I-VI | 2500-5000 | 0-1 | 0.75-1.3 | 100-3000 | 20-200 | >5 or matrix of island spots | -5-10 (1-10 sec) |

Table 5.

Characteristics of flashlamp radiation for pigment lesion treatment

| Skin type | Color temperature of lamp, °K | Light spectrum | | Pulsewidth ms | Fluence, $J/cm^2$ (after filtering) | Beam width, mm | Cooling temperature, °C |
|---|---|---|---|---|---|---|---|
| | | Thickness of water filter, mm | Bands of wavelengths, μm | | | | |
| I-IV | 5000-10000 | 0-3 | 1. 0.36-1.3<br>2. 0.5-1.3<br>3. 0.6-1.3<br>4. 0.36-0.6<br>5. 0.5-0.6 | 0.05-500 | 1-100 | >1 or island of spots | -5-36 (0.3-10 sec) |

EP 1 665 996 A2

Table 6.

Characteristics of flashlamp radiation for dermis treatment for wrinkle improvement

| Skin type | Color temperature of lamp, °K | Light spectrum | | Pulsewidth ms | Fluence, $J/cm^2$ (after filtering) | Beam width, mm | Cooling temperature, °C |
|---|---|---|---|---|---|---|---|
| | | Thickness of water filter, mm | Bands of wavelengths, µm | | | | |
| I-VI | 2500-5000 | 0.1-3 | 1.  0.85-1.85<br>2.  0.85-1.85 & 2.1-2.3 | 10-2000 | 20-200 | >3  or matrix of island spots | -5-10 (0.3-1 sec) |

EP 1 665 996 A2

**Claims**

1. An apparatus for performing a treatment on tissue, comprising:

   a hand piece having a waveguide with an output end for applying radiation from an optical radiation source to the tissue; and
   a mask coupled to the hand piece, the mask having a spatial pattern to allow passage of radiation to provide separate treatment spots in the tissue.

2. Apparatus according to claim 1 wherein the mask is coupled to the output end of the waveguide.

3. Apparatus according to claim 1 or 2 wherein the mask is reflective.

4. Apparatus according to claim 1, 2 or 3 wherein the mask is non-absorptive.

5. Apparatus according to any preceding claim wherein the optical radiation source is an incoherent source.

6. Apparatus according to claim 5 wherein the optical radiation source is a lamp.

7. Apparatus according to any preceding claim wherein the optical radiation source is in the hand piece.

8. Apparatus according to any preceding claim wherein the mask is a coating on the output end of the waveguide.

9. Apparatus according to any preceding claim wherein the apparatus includes a cooling mechanism to cool the tissue during use.

10. Apparatus according to claim 9 wherein the cooling mechanism cools the waveguide.

11. Apparatus according to any preceding claim wherein the spatial pattern includes optical openings.

12. Apparatus according to claim 11 wherein the openings have identical shapes.

13. Apparatus according to claim 12 wherein the openings are shaped as one of squares, rectangles or circles.

14. Apparatus according to claim 11, 12 or 13 wherein the optical openings are periodically spaced in the mask.

15. Apparatus according to any preceding claim further comprising a contact sensor to sense contact of the apparatus with the tissue.

16. Apparatus according to any preceding claim further comprising a mechanism for directing photons reflected from the mask back toward the mask and the tissue.

17. Apparatus according to claim 16 wherein the mechanism includes a reflector.

18. Apparatus according to any preceding claim further comprising a mechanism for directing photons reflected from the tissue back toward the tissue.

19. Apparatus according claim 18 wherein the mechanism comprises a reflector formed on an outer surface of the waveguide.

20. Apparatus according claim 19 wherein the reflector is a scattering reflector.

21. Apparatus according to any preceding claim wherein at least some of the treatment spots in the tissue have a size less than $50\mu$m.

22. Apparatus according to any preceding claim wherein the mask is transmissive in discrete regions.

23. Apparatus according to any preceding claim wherein the mask is transmissive and reflective in regions.

24. Apparatus according to any preceding claim wherein the mask comprises a coating on the waveguide.

25. Apparatus according to any preceding claim wherein the mask is formed by phase modulation of light passing through the waveguide.

26. Apparatus according to any preceding claim wherein the mask is formed by variations in the refractive index of the waveguide.

27. Apparatus according to any preceding claim wherein the mask is formed by surface modulation on the surface of the waveguide.

28. An apparatus utilizing a lamp for treatment of a patient's skin, said apparatus including:

   a waveguide adapted to be in optical contact with the patients skin; and
   a mechanism for directing photons from said lamp through said waveguide to the patient's skin, said mechanism including a submechanism which inhibits the loss of photon from said apparatus.

29. An apparatus as claimed in claim 28 wherein said mechanism includes a reflector, said submechanism including said reflector and waveguide being sized and shaped so that they fit together with substantially no gap therebetween.

30. An apparatus as claimed in claim 29 including a reflective material substantially sealing any gap between said reflector and waveguide.

31. An apparatus as claimed in claim 28 wherein said mechanism includes a reflector, said reflector being sized and mounted with respect to said lamp so as to minimize the number of reflections for each photon on said reflector.

32. An apparatus as claimed in claim 31 wherein said reflector is small enough and mounted close enough to said lamp to achieve said minimum number of reflections.

33. An apparatus as claimed in claim 31 wherein said reflector is formed on an outer surface of said lamp.

34. An apparatus as claimed in claim 31 including a tube surrounding said lamp, there being a gap between said lamp and tube through which a fluid is flowed to cool the lamp.

35. An apparatus as claimed in claim 34 wherein said reflector is formed on one of an inner and outer surface of said tube.

36. An apparatus as claimed in claim 31 wherein said reflector has a substantially cylindrical shape.

37. An apparatus as claimed in claim 31 wherein said reflector is a scattering reflector.

38. An apparatus as claimed in claim 37 including a mechanism for controlling the wavelengths filtered by said scattering reflector.

39. An apparatus as claimed in claim 31 wherein said reflector is of a material which filters selected wavelengths of light from said lamp impinging thereon.

40. An apparatus as claimed in claim 28 wherein said mechanism includes a reflector, wherein there is a gap between said reflector and said waveguide, and including a second reflector in said gap which in conjunction with said reflector directs substantially all photons from said lamp to said waveguide.

41. An apparatus as claimed in claim 28 including a mechanism for selectively filtering light from said lamp to achieve a desired wavelength spectrum, said mechanism for selectively filtering being included as part of at least one of said lamp, a coating formed on said lamp, a tube surrounding said lamp, a filter device in a gap between said lamp and said tube, a reflector for light from said lamp, the waveguide, and a filter device between said lamp and said waveguide.

42. An apparatus as claimed in claim 41 wherein said mechanism for selectively filtering is included as part of a plurality of the components listed in claim 41.

**43.** An apparatus as claimed in claim 41 wherein said mechanism for selectively filtering is at least one of an absorption filter, a selectively reflecting filter, and a spectral resonant scatterer.

**44.** An apparatus as claimed in claim 41 wherein said mechanism includes a multilayer coating.

**45.** An apparatus as claimed in claim 28 wherein said waveguide is of a length selected to enhance uniformity of the light output from said lamp.

**46.** An apparatus as claimed in claim 45 wherein the uniformity of light output from said waveguide has resonances as a function of waveguide length, and wherein the length of said waveguide is equal to one of the resonant lengths.

**47.** An apparatus as claimed in claim 45 wherein said waveguide has a width and depth at an end of the waveguide adjacent the lamp, and wherein the length of the waveguide is much greater then the smaller of said width and depth.

**48.** An apparatus as claimed in claim 28 including a mechanism for controlling the angular spectrum of photons within the patients skin.

**49.** An apparatus as claimed in claim 48 including a gap between the lamp and said waveguide which gap is filled with a substance having a selected index of refraction.

**50.** An apparatus as claimed in claim 49 wherein the length of said gap is minimized.

**51.** An apparatus as claimed in claim 49 wherein said gap is filled with air.

**52.** An apparatus as claimed in claim 28 wherein said waveguide has a larger area at a light receiving surface then at a light output surface, and wherein said waveguide has curved sides between said surfaces.

**53.** An apparatus as claimed in claim 28 wherein said waveguide has a plurality of cuts formed therethrough, said cuts being adapted to have a coolant fluid flow therethrough.

**54.** An apparatus as claimed in claim 28 wherein said waveguide has a surface in contact with the patients skin which is patterned to control the delivery of photons to the patient's skin.

**55.** An apparatus as claimed in claim 28 wherein said waveguide has a surface in contact with the patient's skin which is concave.

**56.** An apparatus as claimed in claim 55 where said waveguide has one of a concave skin contacting surface and a rim surrounding the waveguide with a concave edge.

**57.** An apparatus as claimed in claim 55 wherein the depth of said concave surface is selected to, in conjunction with pressure applied to the apparatus, control the depth of blood vessels treated by the apparatus.

**58.** An apparatus as claimed in claim 57 including a mechanism for detecting the depth of blood vessels in which blood flow is restricted by application of said concave surface under pressure to the patient's skin, said mechanism permitting pressure to be controlled to permit treatment of vessels at a desired depth.

**59.** An apparatus as claimed in claim 28 wherein said waveguide has a skin contacting surface shaped to permit the application of selective pressure to the patient's skin and to thereby control the depth at which treatment is performed.

**60.** An apparatus as claimed in claim 59 wherein said apparatus is being used to treat blood vessels, and including a mechanism for detecting the depth of blood vessels in which blood flow is restricted by application of said surface under pressure to the patient's skin and to thereby control the depth at which treatment is performed.

**61.** An apparatus as claimed in claim 28 wherein said waveguide is at least in part one of a lasing and a superluminescent waveguide.

**62.** An apparatus as claimed in claim 61 wherein said waveguide includes a lasing waveguide inside an optical waveguide.

63. An apparatus as claimed in claim 28 wherein said waveguide has a skin contacting surface, and including a mechanism which delivers a cooling spray to both the patient's skin and said skin contacting surface just prior to said surface making contact with the skin.

64. An apparatus as claimed in claim 63 wherein said waveguide includes a lower portion adjacent the patient's skin of a material which is a good conductor of heat and an upper portion of a material which is not a good conductor of heat, the thickness of said lower portion controlling the depth of cooling in the patient's skin.

65. An apparatus as claimed in claim 63 wherein said mechanism includes a detector indicating when the apparatus is within a predetermined distance of the patient's skin, said cooling spray being activated in response to said detector.

66. An apparatus as claimed in claim 28 including a rearward facing light output channel from said waveguide which leads to a backscatter detector, said channel being at an angle □ to a perpendicular to the skin which assures that only backscattered light reaches the detector.

67. An apparatus as claimed in claim 28 wherein said lamp is driven with a power profile which is one of the power profiles 44, 45 and 46 of Fig. 11.

68. An apparatus as claimed in claim 28 wherein said waveguide is formed as a unitary component with said lamp passing through an opening formed therein.

69. A method for utilizing a lamp for performing hair removal utilizing the parameters of table 1.

70. A method for utilizing a lamp for performing treatment of vascular lesions utilizing the parameters of table 2, 3 and 4.

71. A method for utilizing a lamp for performing skin rejuvenation utilizing the parameters of tables 2 and 6.

72. A method for utilizing a lamp for performing treatment of acne by at least one of killing bacteria, thermolysis of the sebaceous gland and killing spider veins feeding the sebaceous gland.

73. A method of utilizing a lamp for performing treatment of pigmented lesions utilizing the parameters of table 5.

74. An apparatus utilizing a lamp for treatment of a patient's skin, said apparatus including:

a waveguide adapted to be in optical contact with the patients skin; and
a mechanism for directing photons from said lamp through said waveguide to the patient's skin, said mechanism including a reflector, said reflector being mounted close enough to said lamp and being small enough so as to minimize the number of reflections for each photon on said reflector.

75. An apparatus as claimed in claim 74 wherein said reflector is formed on an outer surface of said lamp.

76. An apparatus as claimed in claim 74 including a tube surrounding said lamp, there being a gap between said lamp and tube through which a fluid is flowed to cool the lamp.

77. An apparatus as claimed in claim 76 wherein said reflector is formed on one of an inside and an outside surface of said tube.

78. An apparatus as claimed in claim 76 wherein said reflector has a substantially cylindrical shape.

79. An apparatus as claimed in claim 76 wherein said reflector is a scattering reflector.

80. An apparatus as claimed in claim 79 including a mechanism for controlling the wavelengths filtered by said scattering reflector.

81. An apparatus as claimed in claim 76 wherein said reflector is of a material which filters selected wavelengths of light from said lamp impinging thereon.

82. An apparatus utilizing a lamp for treatment of a patient's skin, said apparatus including:

a waveguide adapted to be in optical contact with the patients skin;

a mechanism for directing photons from said lamp through said waveguide to the patient's skin; and

a mechanism for selectively filtering light from said lamp to achieve a desired wavelength spectrum, said mechanism for selectively filtering being included as part of at least one of said lamp, a coating formed on said lamp, a tube surrounding said lamp, a filter device in a gap between said lamp and said tube, a reflector for light from said lamp, the waveguide, and a filter device between said lamp and said waveguide.

83. An apparatus as claimed in claim 82 wherein said mechanism for selectively filtering is included as part of a plurality of the components listed in claim 82.

84. An apparatus as claimed in claim 82 wherein said mechanism for selectively filtering is at least one of an absorption filter, a selectively reflecting filter, and a spectral resonant scatterer.

85. An apparatus as claimed in claim 82 wherein said mechanism includes a multilayer coating.

86. An apparatus utilizing an optical radiation source for treatment of a patient's skin, said apparatus including:

a waveguide adapted to be in optical contact with the patients skin; and

a mechanism for directing photons from said source through said waveguide to the patient's skin, said waveguide being of a length selected enhance uniformity of the optical output from said apparatus.

87. An apparatus as claimed in claim 86 wherein the uniformity of optical output from said waveguide has resonances as a function of waveguide length, and wherein the length of said waveguide is equal to one of the resonant lengths.

88. An apparatus as claimed in claim 86 wherein said waveguide has a width and depth at an end of the waveguide adjacent the source, and wherein the length of the waveguide is much greater then the smaller of said width and depth.

89. An apparatus utilizing a lamp for treatment of a patient's skin, said apparatus including:

a waveguide adapted to be in optical contact with the patients skin;

a mechanism for directing photons from said lamp through said waveguide to the patient's skin; and

a gap between the lamp and said waveguide which gap is filled with a substance having an index of refraction so as to selectively control the angular spectrum of photons within the patient's skin.

90. An apparatus as claimed in claim 89 including a tube spaced from and substantially surrounding said lamp, and wherein said gap is between said tube and said waveguide.

91. An apparatus as claimed in claim 89 wherein the length of said gap is minimized.

92. An apparatus as claimed in claim 89 wherein said gap is filled with air.

93. An apparatus utilizing an optical radiation source for treatment of a patient's skin, said apparatus including:

a waveguide adapted to be in optical contact with the patients skin, said waveguide having a larger area at a radiation receiving surface then at a radiation output surface, and wherein said waveguide has curved sides between said surfaces; and

a mechanism for directing photons from said source through said waveguide to the patient's skin.

94. An apparatus utilizing an optical radiation source for treatment of a patient's skin, said apparatus including:

a waveguide adapted to be in optical contact with the patients skin, said waveguide having a larger area at a radiation receiving surface then at a radiation output surface and having side walls between said surfaces;

a reflector on each of said walls to inhibit photon leakage through said walls; and

a mechanism for directing photons from said source through said waveguide to the patient's skin.

95. An apparatus utilizing an optical radiation source for treatment of a patient's skin, said apparatus including:

a waveguide adapted to be in optical contact with the patients skin, said waveguide having a plurality of cuts formed therethrough, said cuts being adapted to have a coolant fluid flow therethrough; and

a mechanism for directing photons from said source through said waveguide to the patient's skin.

96. An apparatus utilizing an optical radiation source to perform optical dermatology on a patient's skin, said apparatus including:

a waveguide adapted to be in contact with the patients skin, said waveguide having a surface in contact with the patients skin which is patterned to control the delivery of photons to the patient's skin; and

a mechanism for directing photons from said source through said waveguide to the patient's skin.

97. An apparatus utilizing an optical radiation source for treatment of a patient's skin, said apparatus including:

a waveguide adapted to be in optical contact with the patients skin, said waveguide having a surface in contact with the patient's skin which is concave; and

a mechanism for directing photons from said source through said waveguide to the patient's skin.

98. An apparatus as claimed in claim 97 where said waveguide has one of a concave skin contacting surface and a rim surrounding the waveguide with a concave edge.

99. An apparatus as claimed in claim 97 wherein the depth of said concave surface is selected to, in conjunction with pressure applied to the apparatus, control the depth of blood vessels treated by the apparatus.

100. An apparatus as claimed in claim 99 including a mechanism for detecting the depth of blood vessels in which blood flow is restricted by application of said concave surface under pressure to the patient's skin, said mechanism permitting pressure to be controlled to permit treatment of vessels at a desired depth.

101. An apparatus utilizing an optical radiation source for treatment of a patient's skin, said apparatus including:

a waveguide adapted to be in optical contact with the patients skin, said waveguide having a skin contacting surface which is adapted for application of selective pressure to the skin to control the depth of treatment; and

a mechanism for directing photons from said source through said waveguide to the patient's skin.

102. An apparatus as claimed in claim 101 wherein said apparatus is being used to treat blood vessels, and including a mechanism for detecting the depth of blood vessels in which blood flow is restricted by application of said surface under pressure to the patient's skin, said mechanism permitting pressure to be controlled to permit treatment of vessels at a desired depth.

103. An apparatus utilizing an optical radiation source for treatment of a patient's skin, said apparatus including:

a waveguide adapted to be in optical contact with the patients skin, said waveguide being at least in part one of a lasing and a superluminescent waveguide; and

a mechanism for directing photons from said source through said waveguide to the patient's skin.

104. An apparatus as claimed in claim 103 wherein said waveguide includes a lasing material with mirrors on the end inside an optical waveguide.

105. An apparatus for utilizing an optical radiation source for treatment of a patient's skin, said apparatus including:

a waveguide adapted to be in optical contact with a patient's skin;

at least one of a lasing and a superluminescent material surrounding said lamp;

and a mechanism for directing photons from said source through said waveguide to the patient's skin.

106. An apparatus utilizing an optical radiation source for treatment of a patient's skin, said apparatus including:

a waveguide having a skin contacting surface adapted to be in contact with the patients skin;

a mechanism for directing photons from said lamp through said waveguide to the patient's skin; and

a mechanism which delivers a cooling spray to both the patient's skin and said skin contacting surface just prior to said surface making contact with the skin.

**107.** An apparatus as claimed in claim 106 wherein said waveguide includes a lower portion adjacent the patient's skin of a material which is a good conductor of heat and an upper portion of a material which is not a good conductor of heat, the thickness of said lower portion controlling the depth of cooling in the patient's skin.

**108.** An apparatus as claimed in claim 106 wherein said mechanism includes a detector indicating when the apparatus is within a predetermined distance of the patient's skin, said cooling spray being activated in response to said detector.

**109.** An apparatus utilizing an optical radiation source for treatment of a patient's skin, said apparatus including:

a waveguide adapted to be in optical contact with the patients skin;
a mechanism for directing photons from said lamp through said waveguide to the patient's skin; and
a rearward facing light output channel from said waveguide which leads to a backscatter detector, said channel being at an angle □ to a perpendicular to the skin which assures that only backscattered light reaches the detector.

**110.** An apparatus utilizing a lamp for treatment of a patient's skin, said apparatus including:

a waveguide adapted to be in optical contact with the patients skin;
a mechanism for directing photons from said lamp through said waveguide to the patient's skin; and
a lamp driver which drives said lamp with a power profile which is one of the power profiles 44, 45 and 46 of Fig. 11.

**111.** An apparatus utilizing a lamp for treatment of a patient's skin, said apparatus including:

a waveguide adapted to be in optical contact with the patients skin, said waveguide being formed as a unitary component with said lamp passing through an opening formed therein, said waveguide including a mechanism for directing photons from said lamp through said waveguide to the patient's skin.

**112.** A method of using optical radiation to treat a patient's skin, said method including:

applying optical radiation from an optical radiation source through a plate having a first surface in contact with the patient's skin to the skin; and
applying a cooling fluid to a surface of the plate opposite said first surface;
the thickness of said plate being selected to control the depth in the patient's skin to which cooling occurs.

**113.** A method of using optical radiation to treat blood vessels in a patient's skin, the method including:

applying optical radiation from an optical radiation source through a waveguide to the patient's skin, the waveguide having a selectively shaped skin-contacting surface; and
applying a selected pressure to the waveguide, the pressure being sufficient in conjunction with the shape of the waveguide, to substantially remove blood from all blood vessels above vessels on which treatment is to be performed.

**114.** A method as claimed in claim 113 wherein said waveguide has a concave skin-contacting surface, the depth of the concave surface, in conjunction with the applied pressure controlling the depth of blood vessels being treated.

**115.** An apparatus for utilizing optical radiation to treat a patient's skin, the apparatus including:

a source of optical radiation; and
a waveguide through which radiation from the source is applied to the patient's skin, the waveguide having scattering properties which are a function of the temperature of the waveguide, whereby the waveguide may automatically control radiation applied to the patient's skin to compensate for changes in patient skin temperature.

**116.** Apparatus for utilizing an optical radiation from a lamp to treat a patient's skin, the apparatus including:

a mechanism for applying radiation from the lamp to the patient's skin; and
a filtering mechanism which prevent all but at least one band of radiation from the lamp to reach the patients skin, said at least one band being selected such that the temperature at a desired target in the patent's skin to the temperature of the patient's epidermis has a selected value.

**117.** Apparatus as claimed in claim 116 wherein said selected value is greater than one.

**118.** Apparatus as claimed in claim 116 wherein there are a plurality of bands passed by said filtering mechanism.

Fig. 1

Fig. 1a

Fig. 2

Fig. 2a

Fig. 3

Fig. 4

37

24 – (1ms, Tc 9940K)
25 – (5ms, Tc 7528K)
26 – (20ms, Tc 5925K)
27 – (50ms, Tc 5058K)
28 – (100ms, Tc 4448K)
29 – (200ms, Tc 3982K)
30 – (500ms, Tc 3399K)

Fig. 5

Fig. 6a

EP 1 665 996 A2

Fig. 6b

Fig. 7a

Fig. 7b

Fig. 7c

Fig. 8

Fig. 9

Fig. 10

Fig. 11

Fig. 12

Fig. 13

Fig. 14

Fig. 15

5

Fig. 16

Fig. 17

Fig. 18

Fig. 19

Fig. 20a

5

Fig. 20b

1

5

55

Fig. 20c

Fig. 20d

Fig. 20e

a.

1

b.

1

Fig. 21

EP 1 665 996 A2

Fig. 22

a.

b.

Fig. 23

Fig. 24

69

68

70

Fig. 25

Fig. 26

Fig. 27

Fig. 28